# EUROPEAN PATENT APPLICATION

(11) **EP 4 817 527 A1**
(43) Date of publication of application: **07.10.2026**
(21) Application number: 26170789.7
(22) Date of filing: 03.04.2026
(51) Int. Cl.: A61B 6/03, A61B 6/00, A61B 6/40, A61B 6/58

(54) **SYSTEMS AND METHODS FOR CROSS-SCATTERING CORRECTION**

(30) Priority: 03.04.2025 CN 202510421165; 03.04.2025 CN 202510423827
(71) Applicant: Shanghai United Imaging Healthcare Co., Ltd., Shanghai 201807 (CN)
(72) Inventor: LIU, Yanyan, Shanghai, 201807 (CN); FU, Jianwei, China, 201807 (CN)
(74) Representative: Wang, Bo

(57) **Abstract**

A method for cross-scattering correction for a dual-source or multi-source CT device may be provided. The dual-source or multi-source CT device at least includes a first source-detector pair and a second source-detector pair. The method comprises obtaining a detection signal curve of a first detector of the first source-detector pair. The method also comprises determining a first convolution region based on the detection signal curve, and determining estimated cross-scattering signal intensities of a plurality of detector channels of the first detector by performing a convolution operation on the first convolution region using a target kernel. The target kernel characterizes scattering contributions from a second source of the second source-detector pair to the first detector. The method further comprises determining corrected signal intensities of the detector channels by performing a cross-scattering correction on the signal intensities of the detector channels based on the estimated cross-scattering signal intensities of the detector channels.

## Description

### CROSS-REFERENCE TO RELATED APPLICATIONS

This application claims priority of Chinese Patent Application No. 202510421165.4 and Chinese Patent Application No. 202510423827.1, both filed on April 03, 2025, the contents of each of which are incorporated herein by reference.

### TECHNICAL FIELD

The present disclosure relates to the medical imaging field, and in particular, to cross-scattering correction for a dual-source or multi-source computed tomography (CT) device.

### BACKGROUND

With the continuous advancement of medical imaging technology, CT plays an increasingly important role in clinical diagnosis. Within CT imaging technology, a dual-source or multi-source CT device refers to a CT imaging system comprising two or more source-detector pairs. Each source-detector pair includes an X-ray source and a corresponding detector arranged opposite to each other.

For a dual-source or multi-source scan performed by the dual-source or multi-source CT device, cross-scattering is one of the key factors affecting image quality. The cross-scattering refers to the phenomenon where X-ray photons emitted by an X-ray source, after interacting with the scanned subject, generate scattered radiation that is subsequently received by a non-corresponding detector instead of the corresponding detector aligned with the X-ray source. This leads to the mixing of extraneous scattering components into the signals collected by the non-corresponding detector.

Thus, it is desired to provide efficient and accurate cross-scattering correction systems and methods for a dual-source or multi-source CT device.

### SUMMARY

According to yet another aspect of the present disclosure, a method for cross-scattering correction for a dual-source or multi-source computed tomography (CT) device may be provided. The dual-source or multi-source CT device at least includes a first source-detector pair and a second source-detector pair. The method is implemented on a computing device having at least one processor and at least one storage device. The method may comprise obtaining a detection signal curve of a first detector of the first source-detector pair. The detection signal curve is generated based on signal intensities detected by a plurality of detector channels of the first detector at a scanning angle during a dual-source or multi-source scan of a target subject. The method may comprise determine a first convolution region based on the detection signal curve. The method may also comprise determining estimated cross-scattering signal intensities of the plurality of detector channels by performing a convolution operation on the first convolution region using a target kernel, wherein the target kernel characterizes scattering contributions from a second source of the second source-detector pair to the first detector. The method may further comprise determining corrected signal intensities of the plurality of detector channels by performing a cross-scattering correction on the signal intensities of the plurality of detector channels based on the estimated cross-scattering signal intensities of the plurality of detector channels.

In some embodiments, to determine a first convolution region based on the detection signal curve, the method comprises the following operations. The method may comprise determining boundary position features of the detection signal curve, wherein the boundary position features indicate projection boundaries of the target subject on the first detector at the scanning angle. The method may further comprise determining the first convolution region based on the boundary position features and the detection signal curve.

In some embodiments, the boundary position features of the detection signal curve are determined based on a position of a first source of the first source-detector pair relative to the contour of the target subject at the scanning angle.

In some embodiments, the boundary position features of the detection signal curve are determined based on intersection points between the detection signal curve and a horizontal axis.

In some embodiments, the boundary position features of the detection signal curve are determined based on the detection signal curve and an intensity threshold.

In some embodiments, the target kernel is determined by: obtaining primary signal intensities detected by the plurality of detector channels during a first scan of a target phantom, wherein a first source of the first source-detector pair is activated and the second source is disactivated during the first scan; obtaining cross-scattering signal intensities detected by the plurality of detector channels during a second scan of the target phantom, wherein the first source is disactivated and the second source is activated during the second scan; and determining the target kernel based on the primary signal intensities and the cross-scattering signal intensities.

In some embodiments, to determine the target kernel based on the primary signal intensities and the cross-scattering signal intensities, the method may comprise the following operations. The method may comprise determining a second convolution region based on a detection signal curve corresponding to the primary signal intensities. The method may further comprise determining the target kernel by performing a deconvolution operation on the cross-scattering signal intensities based on the second convolution region.

In some embodiments, the method further comprises: determining a size of the target subject along a first direction, wherein the first direction is perpendicular to a second direction connecting a first source of the first source-detector pair and an imaging center at the scanning angle; and determining the target kernel from a plurality of candidate kernels based on the size of the target subject, wherein the plurality of candidate kernels are predetermined based on candidate phantoms having different phantom sizes.

In some embodiments, the method further comprises: determining whether a termination condition is satisfied based on the estimated cross-scattering signal intensities and the signal intensities; in response to determining that the termination condition is not satisfied, designating the corrected signal intensities as the signal intensities and repeating the method of claim 1 until the termination condition is satisfied.

In some embodiments, to determine the corrected signal intensities of the plurality of detector channels, the method may comprise the following operations. The method may comprise, for each of the plurality of detector channels, determining a signal intensity error of the detector channel based on the estimated cross-scattering signal intensity of the detector channel and a mapping relationship between cross-scattering signal intensities and signal intensity errors corresponding to the detector channel; determining a corrected cross-scattering signal intensity of the detector channel based on the signal intensity error and the estimated cross-scattering signal intensity of the detector channel; e determining the corrected signal intensity of the detector channel by performing the cross-scattering correction on the signal intensity of the detector channel based on the corrected cross-scattering signal intensity of the detector channel.

In some embodiments, the mapping relationship corresponding to the detector channel is determined based on a plurality of sets of reference cross-scattering signal intensities detected by the first detector in a plurality of reference scans. The plurality of reference scans are performed on a plurality of phantoms with different sizes or on one phantom at different positions. A first source of the first source-detector pair is disactivated and the second source is activated during the plurality of reference scans.

In some embodiments, the mapping relationship corresponding to the detector channel is determined by: for each set of reference cross-scattering signal intensities, determining a reference signal intensity error and a base cross-scattering signal intensity for the detector channel based on the set of reference cross-scattering signal intensities; and determining the mapping relationship corresponding to the detector channel based on the reference signal intensity error and the base cross-scattering signal intensity corresponding to each set of reference cross-scattering signal intensity.

In some embodiments, to determine a reference signal intensity error and a base cross-scattering signal intensity for the detector channel based on the set of reference cross-scattering signal intensities, the method may comprise the following operations. The method may comprise obtaining a reference detection signal curve corresponding to the set of reference cross-scattering signal intensities. The method may comprise determining a smoothed detection signal curve by performing a smooth operation on the reference detection signal curve. The method may further comprise determining the reference signal intensity error and the base cross-scattering signal intensity of the detector channel based on the reference detection signal curve and the smoothed detection signal curve.

In some embodiments, the reference signal intensity error of the detector channel is determined by: determining a signal intensity difference of the detector channel based on a difference between the reference detection signal curve and the smoothed detection signal curve; and in response to determining that the signal intensity difference satisfies a preset condition, designating the signal intensity difference as the reference signal intensity error of the detector channel.

In some embodiments, to determine a reference signal intensity error and a base cross-scattering signal intensity for the detector channel based on the set of reference cross-scattering signal intensities, the method may comprise the following operations. The method may comprise generating a phantom image based on the set of reference cross-scattering signal intensity. The method may comprise generating a smoothed phantom image by performing a smooth operation on the phantom image. The method may further comprise determining the reference signal intensity error and the base cross-scattering signal intensity of the detector channel based on the phantom image and the smoothed phantom image.

In some embodiments, to determine the reference signal intensity error and the base cross-scattering signal intensity of the detector channel based on the phantom image and the smoothed phantom image, the method may comprise the following operations. The method may comprise determining a difference image between the phantom image and the smoothed phantom image. The method may comprise obtaining forward projection data of the difference image. The method may further comprise determining the reference signal intensity error and the base cross-scattering signal intensity of the detector channel based on the forward projection data.

In some embodiments, the first detector comprises a plurality of detector modules. Within each detector module, the detector channels share a common mapping relationship.

In some embodiments, the method further comprises: for each detector channel, determining the mapping relationship corresponding to the detector channel from a plurality of candidate mapping relationships corresponding to the detector channel based on a tube voltage used in the dual-source or multi-source scan, wherein the plurality of candidate mapping relationships are predetermined based on different tube voltages.

According to another aspect of the present disclosure, a system for cross-scattering correction for a dual-source or multi-source computed tomography (CT) device may be provided. The dual-source or multi-source CT device at least includes a first source-detector pair and a second source-detector pair. The system may include at least one storage device including a set of instructions and at least one processor. The at least one processor may be configured to communicate with the at least one storage device. When executing the set of instructions, the at least one processor may be configured to direct the system to perform one or more of the following operations. The system may obtain a detection signal curve of a first detector of the first source-detector pair, wherein the detection signal curve is generated based on signal intensities detected by a plurality of detector channels of the first detector at a scanning angle during a dual-source or multi-source scan of a target subject. The system may further determine a first convolution region based on the detection signal curve. The system may determine estimated cross-scattering signal intensities of the plurality of detector channels by performing a convolution operation on the first convolution region using a target kernel, wherein the target kernel characterizes scattering contributions from a second source of the second source-detector pair to the first detector. Then, the system may determine corrected signal intensities of the plurality of detector channels by performing a cross-scattering correction on the signal intensities of the plurality of detector channels based on the estimated cross-scattering signal intensities of the plurality of detector channels.

According to yet another aspect of the present disclosure, a non-transitory computer readable medium may be provided. The non-transitory computer readable medium comprises a set of instructions for cross-scattering correction for a dual-source or multi-source computed tomography (CT) device. The dual-source or multi-source CT device at least includes a first source-detector pair and a second source-detector pair. When executed by at least one processor, the set of instructions direct the at least one processor to effectuate a method. The method may comprise obtaining a detection signal curve of a first detector of the first source-detector pair. The detection signal curve is generated based on signal intensities detected by a plurality of detector channels of the first detector at a scanning angle during a dual-source or multi-source scan of a target subject. The method may comprise determine a first convolution region based on the detection signal curve. The method may also comprise determining estimated cross-scattering signal intensities of the plurality of detector channels by performing a convolution operation on the first convolution region using a target kernel, wherein the target kernel characterizes scattering contributions from a second source of the second source-detector pair to the first detector. The method may further comprise determining corrected signal intensities of the plurality of detector channels by performing a cross-scattering correction on the signal intensities of the plurality of detector channels based on the estimated cross-scattering signal intensities of the plurality of detector channels.

According to yet another aspect of the present disclosure, a method for cross-scattering correction for a dual-source or multi-source computed tomography (CT) device may be provided. The dual-source or multi-source CT device includes a plurality of detectors, the plurality of detectors include a first detector having a plurality of detector channels. The method is implemented on a computing device having at least one processor and at least one storage device. The method may comprise: for each of the plurality of detector channels, determining an estimated cross-scattering signal intensity of the detector channel based on signal intensities detected by the plurality of detector channels at a scanning angle during a dual-source or multi-source scan of a target subject; determining a signal intensity error of the detector channel based on the estimated cross-scattering signal intensity of the detector channel and a mapping relationship between cross-scattering signal intensities and signal intensity errors corresponding to the detector channel; determining a corrected cross-scattering signal intensity of the detector channel based on the signal intensity error and the estimated cross-scattering signal intensity of the detector channel; and determining a corrected signal intensity of the detector channel by performing the cross-scattering correction on the signal intensity of the detector channel based on the corrected cross-scattering signal intensity of the detector channel.

According to another aspect of the present disclosure, a system for cross-scattering correction for a dual-source or multi-source computed tomography (CT) device may be provided. The dual-source or multi-source CT device includes a plurality of detectors, the plurality of detectors include a first detector having a plurality of detector channels. The system may include at least one storage device including a set of instructions and at least one processor. The at least one processor may be configured to communicate with the at least one storage device. When executing the set of instructions, the at least one processor may be configured to direct the system to perform one or more of the following operations. The system may, for each of the plurality of detector channels, determine an estimated cross-scattering signal intensity of the detector channel based on signal intensities detected by the plurality of detector channels at a scanning angle during a dual-source or multi-source scan of a target subject. The system may, for each of the plurality of detector channels, determine a signal intensity error of the detector channel based on the estimated cross-scattering signal intensity of the detector channel and a mapping relationship between cross-scattering signal intensities and signal intensity errors corresponding to the detector channel. The system may, for each of the plurality of detector channels, determine a corrected cross-scattering signal intensity of the detector channel based on the signal intensity error and the estimated cross-scattering signal intensity of the detector channel. The system may, for each of the plurality of detector channels, further determine a corrected signal intensity of the detector channel by performing the cross-scattering correction on the signal intensity of the detector channel based on the corrected cross-scattering signal intensity of the detector channel.

According to yet another aspect of the present disclosure, a non-transitory computer readable medium may be provided. The non-transitory computer readable medium comprises a set of instructions for cross-scattering correction for a dual-source or multi-source computed tomography (CT) device. The dual-source or multi-source CT device includes a plurality of detectors, the plurality of detectors include a first detector having a plurality of detector channels. When executed by at least one processor, the set of instructions direct the at least one processor to effectuate a method. The method may comprise: for each of the plurality of detector channels, determining an estimated cross-scattering signal intensity of the detector channel based on signal intensities detected by the plurality of detector channels at a scanning angle during a dual-source or multi-source scan of a target subject; determining a signal intensity error of the detector channel based on the estimated cross-scattering signal intensity of the detector channel and a mapping relationship between cross-scattering signal intensities and signal intensity errors corresponding to the detector channel; determining a corrected cross-scattering signal intensity of the detector channel based on the signal intensity error and the estimated cross-scattering signal intensity of the detector channel; and determining a corrected signal intensity of the detector channel by performing the cross-scattering correction on the signal intensity of the detector channel based on the corrected cross-scattering signal intensity of the detector channel.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1A is a schematic diagram illustrating an exemplary medical system according to some embodiments of the present disclosure;
FIGs. 1B and 1C are schematic diagrams illustrating an exemplary dual-source CT device according to some embodiments of the present disclosure;
FIG. 2 is a schematic diagram illustrating exemplary hardware and/or software components of an exemplary computing device according to some embodiments of the present disclosure;
FIG. 3 is a block diagram illustrating an exemplary processing device according to some embodiments of the present disclosure;
FIG. 4 is a flowchart illustrating an exemplary cross-scattering correction process according to some embodiments of the present disclosure;
FIG. 5 is a schematic diagram illustrating exemplary detection signal curves according to some embodiments of the present disclosure;
FIG. 6 is a flowchart illustrating an exemplary process for determining a first convolution region according to some embodiments of the present disclosure;
FIG. 7A and 7B are schematic diagrams illustrating an exemplary process for determining boundary position features of a detection signal curve according to some embodiments of the present disclosure;
FIG. 8A is a schematic diagram illustrating an exemplary process for determining boundary position features of a detection signal curve according to some embodiments of the present disclosure;
FIG. 8B is a schematic diagram illustrating an exemplary process for determining boundary position features of a detection signal curve according to some embodiments of the present disclosure;
FIG. 9 is a schematic diagram illustrating an exemplary process for determining boundary position features of a detection signal curve according to some embodiments of the present disclosure;
FIG. 10 is a schematic diagram illustrating an exemplary process for adjusting boundary position features according to some embodiments of the present disclosure;
FIGs. 11A-11D are schematic diagrams illustrating an exemplary a dual-source or multi-source scan on a shoulder of the target subject at a first scanning angle according to some embodiments of the present disclosure;
FIGs. 12A-12D are schematic diagrams illustrating an exemplary a dual-source or multi-source scan of a shoulder of the target subject at a second scanning angle according to some embodiments of the present disclosure;
FIG. 13 is a flowchart illustrating an exemplary process for determining a target kernel according to some embodiments of the present disclosure;
FIG. 14 is a schematic diagram illustrating a size of a target subject at a specific scanning angle according to some embodiments of the present disclosure;
FIG. 15 is a flowchart illustrating an exemplary cross-scattering correction process for a dual-source or multi-source scan according to some embodiments of the present disclosure;
FIG. 16 is a flowchart illustrating an exemplary process for determining a mapping relationship according to some embodiments of the present disclosure;
FIG. 17 is a schematic diagram illustrating an exemplary process for determining a reference signal intensity error and a base cross-scattering signal intensity of a detector channel according to some embodiments of the present disclosure;
FIG. 18 is a schematic diagram illustrating exemplary reference detection signal curves according to some embodiments of the present disclosure;
FIG. 19 is a schematic diagram illustrating an exemplary process 1900 for determining a reference signal intensity error and a base cross-scattering signal intensity of a detector channel according to some embodiments of the present disclosure;
FIG. 20 is a schematic diagram illustrating an exemplary process phantom image according to some embodiments of the present disclosure; and
FIG. 21 is a schematic diagram illustrating an exemplary mapping relationship according to some embodiments of the present disclosure.

### DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high level, without detail, in order to avoid unnecessarily obscuring aspects of the present disclosure. Various modifications to the disclosed embodiments will be readily apparent to those skilled in the art, and the general principles defined herein may be applied to other embodiments and applications without departing from the spirit and scope of the present disclosure. Thus, the present disclosure is not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

The term "system," "engine," "unit," "module," and/or "block" used herein are one method to distinguish different components, elements, parts, sections or assemblies of different levels in ascending order. However, the terms may be displaced by another expression if they achieve the same purpose. Generally, the word "module," "unit," or "block," as used herein, refers to logic embodied in hardware or firmware, or to a collection of software instructions. Such modules/units/blocks may be implemented as software and/or hardware and may be stored in any type of non-transitory computer-readable medium or another storage device. In some embodiments, a software module/unit/block may be compiled and linked into an executable program. It will be appreciated that software modules can be callable from other modules/units/blocks or from themselves, and/or may be invoked in response to detected events or interrupts. Software modules/units/blocks configured for execution on computing devices may be provided on a computer-readable medium It will be further appreciated that hardware modules/units/blocks may be included in connected logic components, and/or can be included of programmable units. The modules/units/blocks or computing device functionality described herein may be implemented as software modules/units/blocks, but may be represented in hardware or firmware. In general, the modules/units/blocks described herein refer to logical modules/units/blocks that may be combined with other modules/units/blocks or divided into sub-modules/subunits/sub-blocks despite their physical organization or storage. The description may be applicable to a system, an engine, or a portion thereof.

It will be understood that when a unit, engine, module, or block is referred to as being "on," "connected to," or "coupled to," another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items. The terms "pixel" and "voxel" in the present disclosure are used interchangeably to refer to an element of an image.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

In the present disclosure, a representation of a subject (e.g., an subject, a patient, or a portion thereof) in an image may be referred to as "subject" for brevity. For instance, a representation of an organ, tissue (e.g., a heart, a liver, a lung), or an ROI in an image may be referred to as the organ, tissue, or ROI, for brevity. Further, an image including a representation of a subject, or a portion thereof, may be referred to as an image of the subject, or a portion thereof, or an image including the subject, or a portion thereof, for brevity. Still further, an operation performed on a representation of a subject, or a portion thereof, in an image may be referred to as an operation performed on the subject, or a portion thereof, for brevity. For instance, a segmentation of a portion of an image including a representation of an ROI from the image may be referred to as a segmentation of the ROI for brevity.

Conventional cross-scattering correction approaches for the dual-source or multi-source scan involves the following steps. First, multiple preset phantoms of varying sizes and eccentric positions are scanned to acquire cross-scattering signals under different conditions, thereby establishing a corresponding signal library. During actual imaging of a target subject, its size and eccentric position are matched against those of the preset phantoms. The cross-scattering signal associated with the phantom that most closely matches the target subject in size and eccentric position is selected as the estimated cross-scattering signal for the target subject. The signal of the target subject acquired by the dual-source or multi-source CT device is then corrected based on the estimated cross-scattering signal to obtain a corrected signal of the target subject. However, due to the inherently limited matching accuracy between the target subject and the preset phantoms, the conventional cross-scattering correction approaches fail to achieve an accurate estimation of the cross-scattering signal, thereby reducing the accuracy of the cross-scattering correction.

The cross-scattering correction method may be performed based on estimated cross-scattering information. The estimated cross-scattering information may include cross-scattering distribution information. For example, one or more factors such as the energy, spatial distribution, and intensity of cross-scattering photons received by the detector may be quantitatively estimated, and the resulting cross-scattering distribution information may serve as the estimated cross-scattering information. For illustration purposes, a signal intensity of cross-scattering photons (also referred to as a cross-scattering signal intensity) received the detector is used as an example in the following description.

To address the above-mentioned problems, the present disclosure provides systems and methods cross-scattering correction for a dual-source or multi-source CT device. The dual-source or multi-source CT device at least includes a first source-detector pair and a second source-detector pair. The systems may obtain a detection signal curve of a first detector of the first source-detector pair. The detection signal curve is generated based on signal intensities detected by a plurality of detector channels of the first detector at a scanning angle during a dual-source or multi-source scan of a target subject. The systems may determine a first convolution region based on the detection signal curve. The systems may determine estimated cross-scattering signal intensities of the plurality of detector channels by performing a convolution operation on the first convolution region using a target kernel. The target kernel characterizes scattering contributions from a second source of the second source-detector pair to the first detector. Then, the systems may determine corrected signal intensities of the plurality of detector channels by performing a cross-scattering correction on the signal intensities of the plurality of detector channels based on the estimated cross-scattering signal intensities of the plurality of detector channels.

According to the methods and systems of the present disclosure, the cross-scattering estimation process does not rely on approximate matching using a limited phantom database. Instead, the methods and systems of the present disclosure directly perform scattering estimation of the detector channels based on the detection signal curve corresponding to the current scanned subject (i.e., the target subject) and the first convolution region using the target kernel, thereby ensuring that the estimation results closely match the actual scattering distribution of the target subject and significantly improving correction accuracy. Furthermore, the methods and systems of the present disclosure perform cross-scattering estimation and correction directly in the raw data (i.e., the detection signal curve) without the need for prior image reconstruction, effectively conserving computational resources and substantially increasing processing speed, thereby achieving efficient cross-scattering correction.

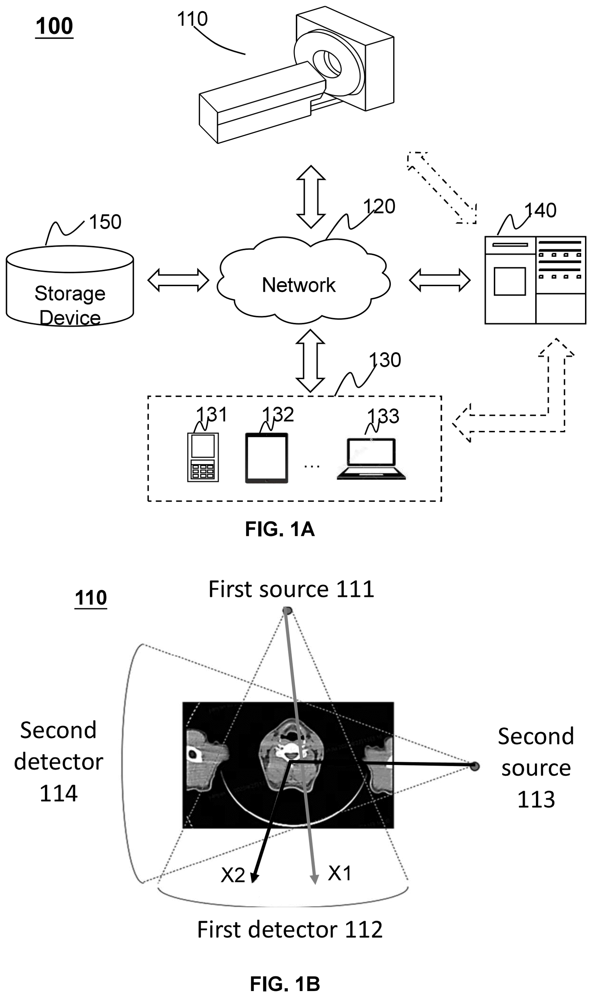

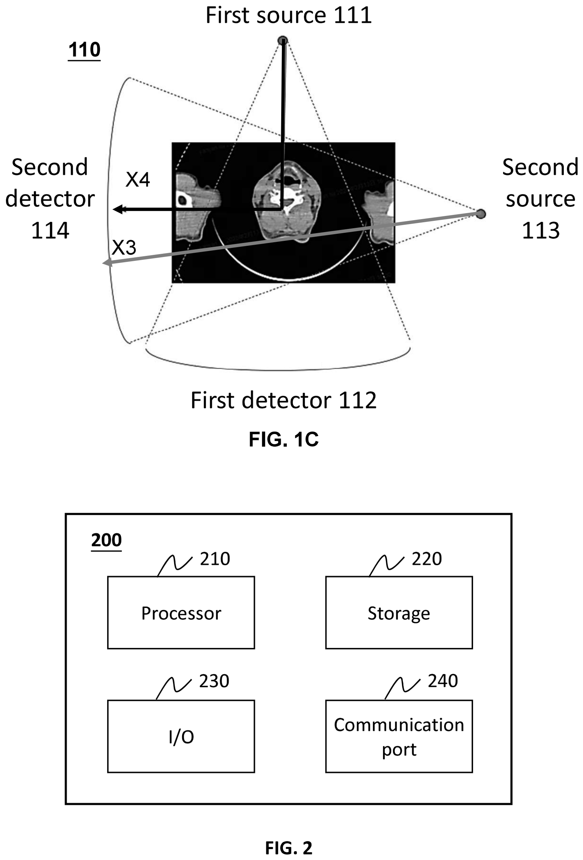

FIG. 1 is a schematic diagram illustrating an exemplary medical system 100 according to some embodiments of the present disclosure. As shown in FIG. 1, the medical system 100 may include an imaging device 110, a network 120, one or more terminals 130, a processing device 140, and a storage device 150. In some embodiments, the imaging device 110, the terminal(s) 130, the processing device 140, and/or the storage device 150 may be connected to and/or communicate with each other via a wireless connection (e.g., the network 120), a wired connection, or a combination thereof. The connection between the components of the medical system 100 may be variable.

The imaging device 110 may be configured to scan a subject (or a part of the subject) to acquire medical image data associated with the subject. The subject may include a biological subject and/or a non-biological subject. For example, the subject may be a human being, an animal, or a portion thereof. As another example, the subject may be a phantom. In some embodiments, the subject may be a patient (or a portion thereof). The medical image data relating to the subject may be used for generating an anatomical image of the subject. The anatomical image may illustrate an internal structure of the subject.

In some embodiments, the imaging device 110 may include a single-modality scanner and/or multi-modality scanner. The single modality scanner may include a CT scanner. The multi-modality scanner may include, for example, a positron emission tomography-computed tomography (PET-CT) scanner, etc. The CT scanner of the single modality scanner and the multi-modality scanner may be a dual-source or multi-source CT device.

The dual-source or multi-source CT device may comprise two or more source-detector pairs. Each source-detector pair includes an X-ray source and a corresponding detector arranged opposite to each other. The X-ray sources are configured to emit X-rays toward the subject, while the detectors are configured to receive the X-rays after they have passed through the subject.

The dual-source or multi-source CT device may be configured to perform a dual-source or multi-source scan on the subject. As used herein, a dual-source or multi-source scan refers to a CT scan by simultaneously activating two or more X-ray sources to scan a subject from different angles, with the respective detectors collecting scan data concurrently.

During the dual-source or multi-source scan performed via the dual-source or multi-source CT device, the signal intensities collected by a detector include primary signal intensities from its corresponding X-ray source. A primary signal intensity refers to the intensity of X-ray signal originating from the detector's corresponding X-ray source. In some occasion, since cross-scattering occurs, the signal intensities collected by the detector further include cross-scattering signal intensities from one or more other X-ray sources other than the corresponding the X-ray source (also referred to as non-corresponding X-ray sources).

For example, FIGs. 1B and 1C are schematic diagrams illustrating an exemplary dual-source CT device 110 according to some embodiments of the present disclosure. As shown in FIGs. 1B and 1C, the dual-source CT device 110 includes a first source-detector pair and a second source-detector pair. The first source-detector pair includes a first source 111 and a corresponding first detector 112 arranged opposite to each other. The second source-detector pair includes a second source 113 and a corresponding second detector 114 arranged opposite to each other.

As shown in FIG. 1B, during a dual-source or multi-source scan performed via the dual-source or multi-source CT device 110, the signal intensities collected by the first detector 112 may include primary signal intensities X1 from the first source 111 and cross-scattering signal intensities X2 from the second source 113. As shown in FIG. 1C, during a dual-source or multi-source scan performed via the dual-source or multi-source CT device 110, the signal intensities collected by the second detector 114 may include primary signal intensities X3 from the second source 113 and cross-scattering signal intensities X4 from the first source 111.

It should be noted that the imaging device 110 described below is merely provided for illustration purposes, and is not intended to limit the scope of the present disclosure.

The network 120 may include any suitable network that can facilitate the exchange of information and/or data for the medical system 100. In some embodiments, one or more components of the medical system 100 (e.g., the imaging device 110, the processing device 140, the storage device 150, the terminal(s) 130) may communicate information and/or data with one or more other components of the medical system 100 via the network 120. For example, the processing device 140 may obtain image data from the imaging device 110 via the network 120.

The terminal(s) 130 may be connected to and/or communicate with the imaging device 110, the processing device 140, and/or the storage device 150. For example, the terminal(s) 130 may display an image of the subject. In some embodiments, the terminal(s) 130 may include a mobile device 131, a tablet computer 132, a laptop computer 133, or the like, or any combination thereof. In some embodiments, the terminal(s) 130 may be part of the processing device 140.

The processing device 140 may process data and/or information obtained from the imaging device 110, the storage device 150, the terminal(s) 130, or other components of the medical system 100. For example, the processing device 140 may obtain image data collected by the imaging device 110 and perform cross-scattering correction on the image data to obtain corrected image data. Further, the processing device 140 may generate a medical image of the subject based on the corrected image data.

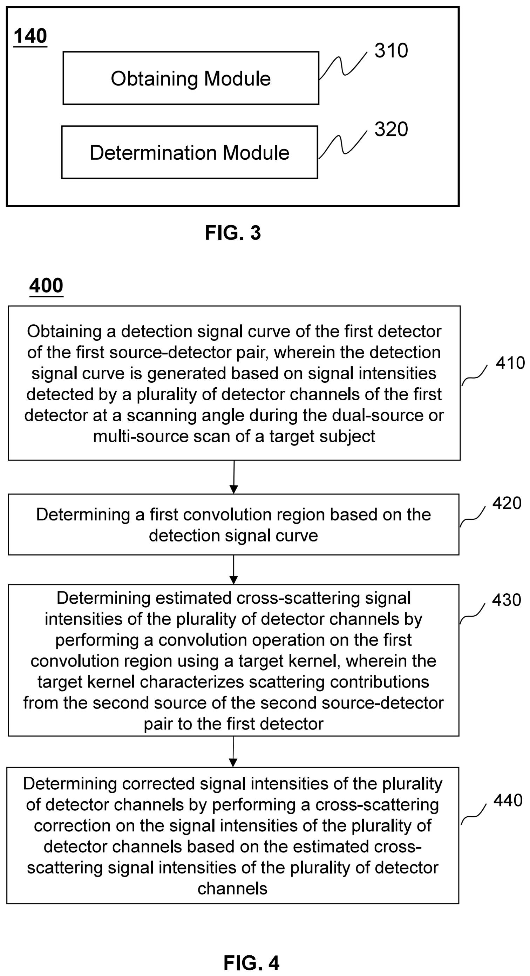

In some embodiments, the processing device 140 (e.g., one or more modules illustrated in FIG. 3) may execute instructions and may accordingly be directed to perform one or more processes (e.g., processes 400, 600, 1300, 1500, and 1600) described in the present disclosure. For example, each of the one or more processes may be stored in a storage device (e.g., the storage device 150) as a form of instructions, and invoked and/or executed by the processing device 140.

In some embodiments, the processing device 140 may be a single server or a server group. In some embodiments, the processing device 140 may be local to or remote from the medical system 100. Merely for illustration, only one processing device 140 is described in the medical system 100. However, it should be noted that the medical system 100 in the present disclosure may also include multiple processing devices. Thus operations and/or method steps that are performed by one processing device 140 as described in the present disclosure may also be jointly or separately performed by the multiple processing devices. For example, if in the present disclosure the processing device 140 of the medical system 100 executes both process A and process B, it should be understood that the process A and the process B may also be performed by two or more different processing devices jointly or separately in the medical system 100 (e.g., a first processing device executes process A and a second processing device executes process B, or the first and second processing devices jointly execute processes A and B).

The storage device 150 may store data, instructions, and/or any other information. In some embodiments, the storage device 150 may store data obtained from the processing device 140, the terminal(s) 130, and/or the imaging device 110. For example, the storage device 150 may store image data collected by the imaging device 110. As another example, the storage device 150 may store target kernels for cross-scattering correction. In some embodiments, the storage device 150 may store data and/or instructions that the processing device 140 may execute or use to perform exemplary methods described in the present disclosure.

It should be noted that the above description of the medical system 100 is intended to be illustrative, and not to limit the scope of the present disclosure. Many alternatives, modifications, and variations will be apparent to those skilled in the art. The features, structures, methods, and other characteristics of the exemplary embodiments described herein may be combined in various ways to obtain additional and/or alternative exemplary embodiments. For example, the medical system 100 may include one or more additional components. Additionally or alternatively, one or more components of the medical system 100 described above may be omitted. As another example, two or more components of the medical system 100 may be integrated into a single component.

FIG. 2 is a schematic diagram illustrating exemplary hardware and/or software components of an exemplary computing device according to some embodiments of the present disclosure. In some embodiments, the processing device 140 may be implemented on the computing device 200. As illustrated in FIG. 2, the computing device 200 may include a processor 210, a storage 220, an input/output (I/O) 230, and a communication port 240.

The processor 210 may execute computer instructions (program code) and perform functions of the processing device 140 in accordance with techniques described herein. The computer instructions may include routines, programs, subjects, components, signals, data structures, procedures, modules, and functions, which perform particular functions described herein. Merely for illustration purposes, only one processor is described in the computing device 200. However, it should be noted that the computing device 200 in the present disclosure may also include multiple processors, and thus operations of a method that are performed by one processor as described in the present disclosure may also be jointly or separately performed by the multiple processors.

The storage 220 may store data/information obtained from the imaging device 110, the terminal device 130, the storage device 150, or any other component of the medical system 100. In some embodiments, the storage 220 may include a mass storage device, a removable storage device, a volatile read- and-write memory, a read-only memory (ROM), or the like, or any combination thereof. In some embodiments, the storage 220 may store one or more programs and/or instructions to perform exemplary methods described in the present disclosure.

The I/O 230 may input or output signals, data, or information. In some embodiments, the I/O 230 may enable user interaction with the processing device 140. In some embodiments, the I/O 230 may include an input device and an output device.

The communication port 240 may be connected to a network (e.g., the network 120) to facilitate data communications. The communication port 240 may establish connections between the processing device 140 and the imaging device 110, the terminal device 130, or the storage device 150. The connection may be a wired connection, a wireless connection, or combination of both that enables data transmission and reception.

It should be noted that the above description of the computing device 200 is merely provided for the purposes of illustration, and not intended to limit the scope of the present disclosure. For persons having ordinary skills in the art, multiple variations and modifications may be made under the teachings of the present disclosure.

FIG. 3 is a block diagram illustrating exemplary processing device 140 according to some embodiments of the present disclosure.

As shown in FIG. 3, the processing device 140 may include an obtaining module 310 and a determination module 320.

The obtaining module 310 may be configured to obtain information relating to the medical system 100. For example, the obtaining module 310 may obtain a detection signal curve of the first detector of the first source-detector pair, wherein the detection signal curve is generated based on signal intensities detected by a plurality of detector channels of the first detector at a scanning angle during the dual-source or multi-source scan of a target subject.. More descriptions regarding the obtaining of the detection signal curve of the first detector may be found elsewhere in the present disclosure. See, e.g., operation 410 in FIG. 4, and relevant descriptions thereof.

The determination module 320 may be configured to determine a first convolution region based on the detection signal curve. More descriptions regarding the determination of the first convolution region may be found elsewhere in the present disclosure. See, e.g., operation 420 in FIG. 4, and relevant descriptions thereof.

The determination module 320 may be also configured to determine estimated cross-scattering signal intensities of the plurality of detector channels by performing a convolution operation on the first convolution region using a target kernel, wherein the target kernel characterizes scattering contributions from the second source of the second source-detector pair to the first detector. More descriptions regarding the determination of the estimated cross-scattering signal intensities may be found elsewhere in the present disclosure. See, e.g., operation 430 in FIG. 4, and relevant descriptions thereof.

The determination module 320 may be also configured to determine corrected signal intensities of the plurality of detector channels by performing a cross-scattering correction on the signal intensities of the plurality of detector channels based on the estimated cross-scattering signal intensities of the plurality of detector channels. More descriptions regarding the determination of the corrected signal intensities of the plurality of detector channels may be found elsewhere in the present disclosure. See, e.g., operation 440 in FIG. 4, and relevant descriptions thereof.

It should be noted that the above description is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, any one of the modules may be divided into two or more units. For instance, the obtaining module 310 may be divided into two units configured to acquire different data. In some embodiments, the processing device 140 may include one or more additional modules, such as a storage module (not shown) for storing data.

FIG. 4 is a flowchart illustrating an exemplary cross-scattering correction process 400 for a dual-source or multi-source scan according to some embodiments of the present disclosure.

During a dual-source or multi-source scan, the dual-source or multi-source CT device rotates to acquire image data from multiple scanning angles for image reconstruction. The scanning angle refers to any angular position of a gantry of the dual-source or multi-source CT device during the dual-source or multi-source scan, at which raw image data (e.g., projection data) is acquired. At each scanning angle, cross-scattering may occur. The process 400 is performed separately for each scanning angle and for each source-detector pair that is affected by cross-scattering. For illustration purposes, the process 400 for performing cross-scattering correction on data acquired by a specific source-detector pair at a single scanning angle is described.

The dual-source or multi-source scan may be performed by the dual-source or multi-source CT device of the imaging device 110. The dual-source or multi-source CT device at least includes a first source-detector pair and a second source-detector pair. The first source-detector pair refers to a source-detector pair of the dual-source or multi-source CT device that is affected by cross-scattering from the second source-detector pair. The second source-detector pair refers to a source-detector pair of the dual-source or multi-source CT device that causes cross-scattering effects on the first source-detector pair. During the dual-source or multi-source scan, the signal intensities collected by a first detector of the first source-detector pair include primary signal intensities from a first source of the first source-detector pair and cross-scattering signal intensities from a second source of the second source-detector pair.

In 410, the processing device 140 (e.g., the obtaining module 310) may obtain a detection signal curve of the first detector of the first source-detector pair, wherein the detection signal curve is generated based on signal intensities detected by a plurality of detector channels of the first detector at a scanning angle during the dual-source or multi-source scan of a target subject.

The first detector may include one or more detector channels. Each detector channel may correspond to one or more detection units. Each detector channel is configured to output a signal intensity based on the X-ray signals detected by its detection units. The scanning angle may be any scanning angle within a scanning angle range of the dual-source or multi-source CT device during the dual-source or multi-source scan. The scanning angle range may be set manually by a user (e.g., an engineer) according to an experience value or determined based on scanning requirements of the target subject.

The detection signal curve reflects, as a function of detector channels, the distribution of the signal intensities detected by the first detector at the scanning angle during the dual-source or multi-source scan. In some embodiments, the detection signal curve is defined in a coordinate system where the horizontal axis represents the detector channel index and the vertical axis represents the signal intensity (or referred to the signal amplitude). This curve is obtained by plotting the signal intensity received by each detector channel against their respective channel indices.

In some embodiments, the processing device 140 may obtain the detection signal curve from one or more components of the medical system 100 (e.g., the storage device 150, the terminals(s) 130) or an external source via a network (e.g., the network 120). For example, the detection signal curve may be generated previously by a computing device (e.g., the processing device 140), and stored in the storage device 150. The processing device 140 may access the storage device 150 and retrieve the detection signal curve.

In 420, the processing device 140 (e.g., the determination module 320) may determine a first convolution region based on the detection signal curve.

During CT imaging, the primary signal intensity varies significantly, directly reflecting attenuation characteristics of the scanned subject along the X-ray path. In contrast, although cross-scattering signals are related to the density of the scanned subject, their spatial distribution (e.g., a range of detector channels affected by the cross-scattering signals) is primarily determined by the geometric shape and boundary features of the scanned subject. In essence, the occurrence and spread of cross-scattering signals are determined more by the spatial extent where the scanned subject intercepts the X-ray beam (i.e., its boundaries), rather than by the internal density distribution of the scanned subject. Therefore, when estimating the spatial distribution of cross-scattering signals, the boundary information of the subject is more critical than the internal density distribution. Specifically, the boundary information primarily determines the range of the detection signal curve along the detector channel dimension (i.e., the horizontal axis), whereas the internal density distribution mainly affects the amplitude of the detection signal curve along the signal intensity dimension (i.e., the vertical axis). Consequently, in cross-scattering signal estimation, greater emphasis should be placed on the detection signal curve's extent along the horizontal axis, while the amplitude variations along the vertical axis can be largely neglected.

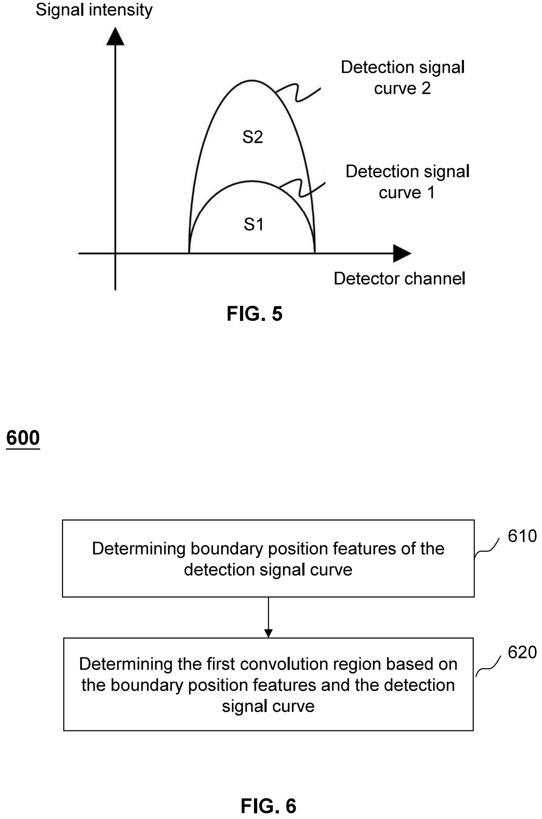

For example, FIG. 5 is a schematic diagram illustrating exemplary detection signal curves according to some embodiments of the present disclosure. The detection signal curve 1 and detection signal curve 2 are obtained by scanning two subjects with similar shapes and dimensions but different attenuation characteristics. As shown in FIG. 5, the detection signal curve 1 and detection signal curve 2 cover the same range of detector channels, yet exhibit significant differences in the signal intensity dimension (i.e., the vertical axis). If a region enclosed by a detection signal curve and the horizontal axis (e.g., a region S1 enclosed by the detection signal curve 1 and the horizontal axis, or a region S2 enclosed by the detection signal curve 12 and the horizontal axis) is determined as a convolution region for convolution operation, the drastic variations in the signal intensity dimension would not only increase computational complexity but also potentially introduce interference due to their low contribution to scatter estimation, thereby affecting accuracy of the scatter estimation.

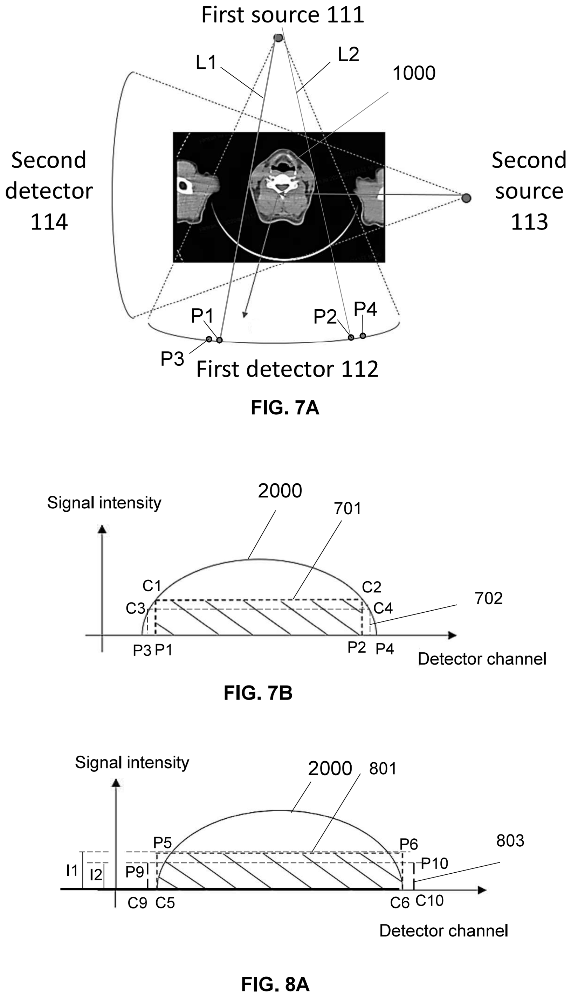

Based on the above principle, in some embodiments, the processing device 140 may determine the first convolution region based on the boundary position features of the detection signal curve and the detection signal curve. As shown in FIGs. 7B, 8A, 8B, and 9A, the first convolution region may be a rectangular convolution region. The boundary position features indicate projection boundaries of the target subject on the first detector at the scanning angle. More descriptions regarding the determination of the first convolution region may be found elsewhere in the present disclosure (e.g., FIG. 6 and the descriptions thereof).

In some embodiments, for different scans, when boundary position features of scanned subjects are the same, the first convolution regions corresponding to the scans may the same. For example, as shown in FIG. 5, although the signal intensity variations of the detection signal curves 1 and 2 differ significantly, the first convolution regions respectively determined for the detection signal curves 1 and 2 may be the same or highly similar due to the same boundary position features.

According to some embodiments of the present disclosure, the first convolution region may be determined based on boundary position features, thereby reducing interference from the signal intensity dimension. Moreover, the region enclosed by the detection signal curve and the horizontal axis may be simplified into a rectangular convolution region defined by boundary position features, substantially reducing the complexity of subsequent convolution operations, thereby conserving computational resources while significantly improving correction efficiency.

In 430, the processing device 140 (e.g., the determination module 320) may determine estimated cross-scattering signal intensities of the plurality of detector channels by performing a convolution operation on the first convolution region using a target kernel, wherein the target kernel characterizes scattering contributions from the second source of the second source-detector pair to the first detector.

The estimated cross-scattering signal intensity of a detector channel may characterize an intensity of a cross-scattering signal received by the detector channel from the second source.

The convolution operation may mathematically simulate how the scattering contribution from the second source spreads across the detector channels of the first detector. In some embodiments, the processing device 140 may perform the convolution operation on the first convolution region using the target kernel to generate a cross-scattering signal curve. The cross-scattering signal curve is defined in a coordinate system where the horizontal axis represents the detector channel index and the vertical axis represents the cross-scattering signal intensities. This curve is obtained by plotting the cross-scattering signal intensity received by each detector channel against their respective channel indices. The detection signal curve characterizes the cross-scattering signal intensities received by the detector channels of the first detector corresponding to the first convolution region. The cross-scattering signal intensities received by the remaining detector channels of the first detector other than the detector channels corresponding to the first convolution region may be zero or approximately zero, i.e., these remaining detector channels are minimally affected or unaffected by cross-scattering from the second source.

An incident X-ray can be considered as a straight line. However, after scattering within a scanned subject, it may irradiate a small surrounding region. A convolution kernel can be used to characterize the range that the scattered photons can reach. By modeling the spatial propagation of scattering through a convolution kernel, the physical scattering process is transformed into operations such as neighborhood-weighted summation in signal processing, thereby enabling the estimation of scattering information.

In some embodiments, the target kernel may be a function or matrix predetermined via a deconvolution method, and the target kernel is used for performing the convolution operation with the first convolution region to simulate the propagation and distribution process of cross-scattering signals.

Since the cross-scattering effects between different source-detector pairs vary, for the first detector, a specific convolution kernel may be predetermined for each non-corresponding X-ray source. The convolution kernel corresponding to an X-ray source characterizes the scattering contributions from the X-ray source to the first detector.

In some embodiments, the convolution kernels corresponding to one or more non-corresponding X-ray sources of the first detector may be generated previously by a computing device (e.g., the processing device 140), and stored in the storage device 150. The processing device 140 may access the storage device 150 and retrieve the target kernel corresponding to the second source. For example, the convolution kernels and the corresponding X-ray sources may be stored in a lookup table. The processing device 140 may obtain the target kernel according to the second source by retrieving the lookup table.

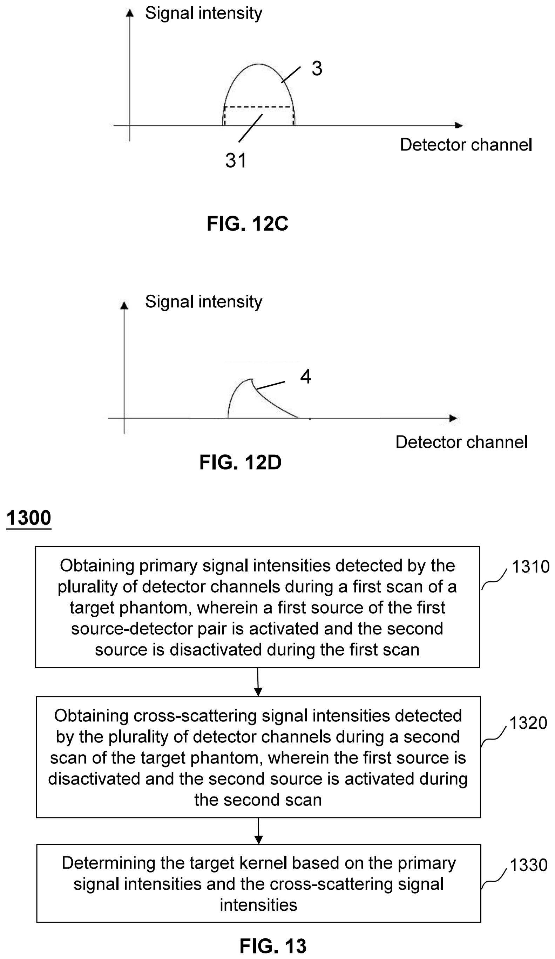

In some embodiments, the target kernel is determined by performing the following operations. Primary signal intensities detected by the plurality of detector channels during a first scan of a target phantom may be obtained. The first source of the first source-detector pair is activated and the second source is disactivated during the first scan. Cross-scattering signal intensities detected by the plurality of detector channels during a second scan of the target phantom may be obtained. The first source is disactivated and the second source is activated during the second scan. Further, the target kernel may be determined based on the primary signal intensities and the cross-scattering signal intensities. More descriptions regarding the determination of the target kernel may be found elsewhere in the present disclosure (e.g., FIG. 13 and the descriptions thereof).

As described above, the spatial distribution of the cross-scattering signals primarily depends on the geometric shape and boundary features of the target subject. In some occasions, the target subject may have an irregular shape. The size of the portion of the target subject radiated by the first source along a direction particular to the radiation direction of the X-ray may vary at different scanning angles, leading to differences in the distribution of the cross-scattering signals. To estimate the cross-scattering signals more accurately, in some embodiments, the processing device 140 may determine the target kernel from a plurality of candidate kernels based on a size of the target subject along the direction particular to the radiation direction of the X-ray at the current scanning angle.

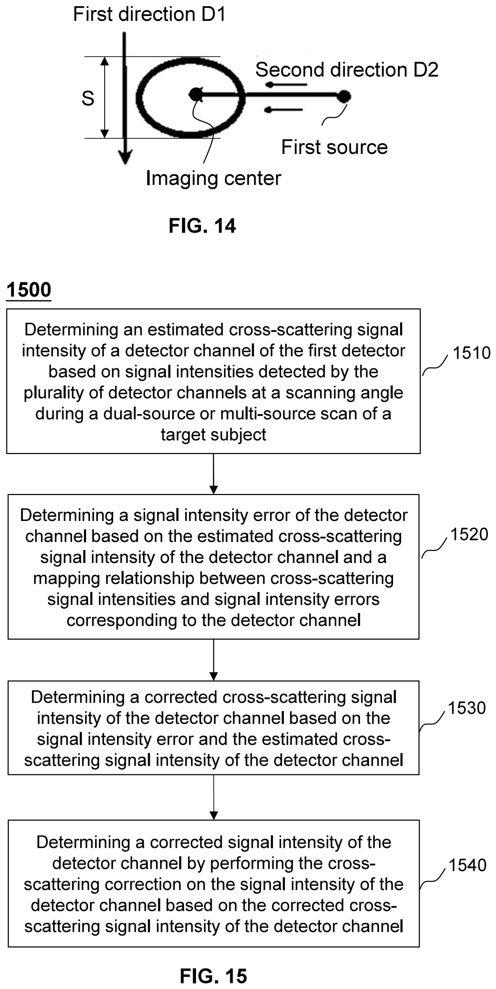

Specifically, the processing device 140 may determine a size of the target subject along a first direction. The first direction is perpendicular to a second direction connecting the first source of the first source-detector pair and an imaging center at the scanning angle. For example, FIG. 14 is a schematic diagram illustrating a size of a target subject at a specific scanning angle according to some embodiments of the present disclosure. As shown in FIG. 14A, at the specific scanning angle, the X-ray irradiates the target subject from a second direction D2, which is a direction connecting the first source and an imaging center at the scanning angle (also referred to as the radiation direction of the X-ray). The processing device 140 may determine a size S of the target subject along a first direction D1. The first direction D1 is perpendicular to the second direction D2.

Further, the processing device 140 may determine the target kernel from a plurality of candidate kernels based on the size of the target subject along the first direction. The plurality of candidate kernels are predetermined based on candidate phantoms having different phantom sizes. The candidate phantoms may have attenuation characteristics similar to those of the target subject to ensure that the generated candidate kernels accurately reflect the cross-scattering characteristics of the target subject. In some embodiments, the candidate phantoms may have material compositions similar to those of the target subject. For instance, when the target subject is a human patient, water phantoms are commonly employed as suitable candidate phantoms due to the fact that the X-ray attenuation properties of water closely approximate those of human soft tissue.

In some embodiments, the plurality of candidate kernels may be determined in a similar manner as how to determine the target kernel described in FIG. 13. In some embodiments, the plurality of candidate kernels may be generated previously by a computing device (e.g., the processing device 140), and stored in the storage device 150. The processing device 140 may select a candidate kernel having a size closest to the size of the target subject from the storage device 150 according to the size of the target subject along the first direction at the scanning angle, and designate the selected candidate kernel as the target kernel.

Merely by way of example, candidate phantoms having diameters of 180 mm and 320 mm may be provided, and two corresponding candidate kernels may be pre-generated. When the size of the target subject along the first direction at the scanning angle is close to 180 mm (e.g., 175 mm), the candidate kernel corresponding to the 180 mm may be determined as the target kernel. When the size of the target subject along the first direction at the scanning angle is close to 320 mm (e.g., 310 mm), the candidate kernel corresponding to the 320 mm may be determined as the target kernel.

Since the size of the target subject along the first direction may reflect the spatial range where the target subject blocks X-rays at the scanning angle, the size of the target subject along the first direction can effectively characterize the range of detector channels over which the cross-scattering signals may spread. By pre-generating candidate kernels corresponding to the candidate phantoms of different sizes (e.g., water phantoms with different diameters), the most matching candidate kernel can be dynamically selected during the dual-source or multi-source scan based on the size of the target subject in the first direction at the current scanning angle, thereby improving the accuracy of scattering estimation.

In 440, the processing device 140 (e.g., the determination module 320) may determine corrected signal intensities of the plurality of detector channels by performing a cross-scattering correction on the signal intensities of the plurality of detector channels based on the estimated cross-scattering signal intensities of the plurality of detector channels.

In some embodiments, for each detector channel, the processing device 140 may determine the corrected signal intensity of the detector channel by adjusting the signal intensity detected by the detector channel based on the estimated cross-scattering signal intensity of the detector channel. For example, the processing device 140 may determine the corrected signal intensity of the detector channel by subtracting the estimated cross-scattering signal intensity from the signal intensity detected by the detector channel. That is, the corrected signal intensity of the detector channel may be determined according to Equation (1) as below: ${I}_{corrected} \left(ch\right) = {I}_{original} \left(ch\right) - {I}_{estimated_cross} \left(ch\right) ,$ (1) where, *I*_{corrected}(*ch*) represents the corrected signal intensity of the detector channel, *I*_{original}(*ch*) represents the signal intensity detected by the detector channel, and *I*_{estimated_cross}(*ch*) represents the estimated cross-scattering signal intensity of the detector channel.

In some embodiments, the second convolution region described in FIG. 13, used for determining the target kernel is determined based on a detection signal curve that does not contain cross-scattering signal intensities, whereas the first convolution region is determined based on the detection signal curve that contains both primary signal intensities and cross-scattering signal intensities. Therefore, when the cross-scattering signal intensities are relatively high, they may have a certain impact on the accuracy of the first convolution region, thereby affecting the accuracy of cross-scattering estimation.

To address this issue, in some embodiments, an iterative correction approach may be adopted to progressively improve the accuracy of cross-scattering estimation. Specifically, the processing device 140 may determine whether a termination condition is satisfied based on the estimated cross-scattering signal intensities and the signal intensities. In response to determining that the termination condition is satisfied, the processing device 140 may terminate the cross-scattering correction process and designate the corrected signal intensities as signals for image reconstruction. In response to determining that the termination condition is not satisfied, the processing device 140 may designate the corrected signal intensities as the signal intensities described in operation 410 and repeat the process 400 until the termination condition is satisfied. In other words, an iterative process including one or more iterations may be performed. In each iteration, operations 410-440 in the process 400 may be performed.

In some embodiments, the termination condition may be determined based on a ratio of the estimated cross-scattering signal intensities to the signal intensities in the current iteration (also referred to as the current signal intensities). For example, for each detector channel, the ratio of the estimated cross-scattering signal intensity of detector channel to the current signal intensity of detector channel may be determined. The processing device 140 may determine whether a termination condition is satisfied based on the ratios of the detector channels. For example, when the ratios of all detector channels are less than a first preset threshold (e.g., 5% or 10%), the processing device 140 may determine that the termination condition is satisfied. When the ratios of one or more detector channels are not less than the first preset threshold, the processing device 140 may determine that the termination condition is not satisfied. As another example, when an average of the ratios of detector channels is less than a second preset threshold (e.g., 4% or 6%), the processing device 140 may determine that the termination condition is satisfied. When the average of the ratios of detector channels is not less than the second preset threshold, the processing device 140 may determine that the termination condition is not satisfied. The first preset threshold and the second preset threshold may be set manually by a user (e.g., an engineer) according to an experience value or a default setting of the medical system 100.

In other embodiments, the termination condition may also be determined based on the number of iterations. A maximum number of iterations (e.g., 3 or 5 iterations) may be previously preset. When the maximum number is reached, the processing device 140 may determine that the termination condition is satisfied. When the maximum number is not reached, the processing device 140 may determine that the termination condition is not satisfied.

Through the above iterative process, the first convolution region may be re-determined in each iteration based on the updated signal intensities (i.e., signals from which a portion of the cross-scattering component has been removed), and the cross-scattering signal intensities may be re-estimated accordingly, thereby progressively approaching the true cross-scattering distribution. This significantly improves the accuracy of cross-scattering estimation, thereby enhancing the quality of the image reconstructed based on the corrected signal intensities.

In some embodiments, for each of the plurality of detector channels, the processing device 140 may determine a corrected cross-scattering signal intensity of the detector channel by performing a correction operation on the estimated cross-scattering signal intensity of the detector channel, and further determine the corrected signal intensity of the detector channel by performing the cross-scattering correction on the signal intensity of the detector channel based on the corrected cross-scattering signal intensity of the detector channel. Specifically, for each of the plurality of detector channels, the processing device 140 may determine a signal intensity error of the detector channel based on the estimated cross-scattering signal intensity of the detector channel and a mapping relationship between cross-scattering signal intensities and signal intensity errors corresponding to the detector channel. Further, the processing device 140 may determine the corrected cross-scattering signal intensity of the detector channel based on the signal intensity error and the estimated cross-scattering signal intensity of the detector channel. Then, the processing device 140 may determine the corrected signal intensity of the detector channel by performing the cross-scattering correction on the signal intensity of the detector channel based on the corrected cross-scattering signal intensity of the detector channel. More descriptions regarding the correction operation on the estimated cross-scattering signal intensity and the determination of the corrected signal intensity based on the corrected cross-scattering signal intensity may be found elsewhere in the present disclosure (e.g., operations 1520-1540 in FIG. 15 and the descriptions thereof).

In some embodiments, the corrected signal intensities may be converted into corrected project data. In some embodiments, after the corrected signal intensities of each detector at each scanning angle are obtained by performing cross-scattering correction on the signal intensities acquired by each detector at each scanning angle, a CT image of the target subject corresponding to the detector may be reconstructed based on the corrected signal intensities of each detector at each scanning angle using an image reconstruction algorithm (e.g., a filtered back projection algorithm or an iterative reconstruction algorithm).

In some embodiments, different source-detector pairs may employ X-rays with different energy levels for scanning. For example, in a dual-source CT scan, the first source may use low-energy rays (e.g., 80 kV) for scanning, while the second source may use high-energy rays (e.g., 140 kV). After performing the aforementioned cross-scattering correction, the corrected signal intensities of the first detector and the corrected signal intensities of the second detector may be obtained respectively. A low-energy image may be reconstructed based on the corrected signal intensities of the first detector and a high-energy image may be reconstructed based on the corrected signal intensities of the second detector. In some embodiments, an image fusion operation may be performed on the low-energy image and the high-energy image to obtain a fused image.

It should be noted that the above-described image reconstruction and post-processing procedures are merely illustrative. In practical applications, corresponding processing methods may be selected based on specific scanning protocols and clinical requirements, and the present disclosure does not impose limitations in this regard.

As described elsewhere in the present disclosure, due to the inherently limited matching accuracy between the target subject and the preset phantoms, the conventional cross-scattering correction approaches fails to achieve an accurate estimation of the cross-scattering signal, thereby reducing the accuracy of the cross-scattering correction.

Compared with the cross-scattering correction approaches, the cross-scattering estimation process in FIG. 4 does not rely on approximate matching using a limited phantom database. Instead, the process 400 directly perform scattering estimation of the detector channels based on the detection signal curve of the target subject and the first convolution region using the target kernel, thereby ensuring that the estimation results closely match the actual scattering distribution of the target subject and significantly improving correction accuracy. Furthermore, the process 400 performs cross-scattering estimation and correction directly in the raw data (i.e., the detection signal curve) without the need for prior image reconstruction, effectively conserving computational resources and substantially increasing processing speed, thereby achieving efficient cross-scattering correction.

Moreover, due to the varying impact of cross-scattering on different detector channels, the process 400 performs cross-scattering correction independently for each detector channel, thereby accurately capturing signal variations among different detector channels and providing a more precise basis for subsequent image reconstruction.

FIG. 6 is a flowchart illustrating an exemplary process 600 for determining a first convolution region according to some embodiments of the present disclosure. In some embodiments, the process 600 may be performed to achieve at least part of operation 420 as described in connection with FIG. 4.

In 610, the processing device 140 may determine boundary position features of the detection signal curve.

The boundary position features indicate, at the scanning angle, the projection boundaries of the target subject on the first detector. As used herein, the projection of the target subject on the first detector refers to a shadow cast by the target subject onto the first detector along the radiation direction of the first source. The projection boundaries define the start and end positions of the projection along the ordered sequence of detector channels (i.e., the horizontal axis of the detection signal curve). In other words, the boundary position features indicate points on the detection signal curve corresponding to the first and last detector channels onto which the target subject is projected.

In some embodiments, the boundary position features of the detection signal curve are determined based on a position of the first source of the first source-detector pair relative to the contour of the target subject at the scanning angle. In some embodiments, the position of the first source may be a focal spot position of the first source. Specifically, the processing device 140 may determine two tangent lines connecting the position of the first source and the contour of the target subject. Further, the processing device 140 may determine two intersection positions where the two tangent lines intersect the first detector (also referred to as theoretical projection boundaries), and designate the detector channels corresponding to the two intersection positions as boundary detector channels. In some embodiments, considering the presence of cross-scattering signals, some detector channels near the theoretical projection boundaries may also receive signals. Therefore, the theoretical projection boundaries may be extended toward two ends of the first detector by a certain number of channels, and the detector channels corresponding to the extended projection boundaries may be designated as the boundary detector channels. Then, the processing device may designate two points on the detection signal curve whose horizontal coordinates correspond to the boundary detector channels as the boundary position features of the detection signal curve.

For example, FIG. 7A and 7B are schematic diagrams illustrating an exemplary process for determining boundary position features of a detection signal curve according to some embodiments of the present disclosure. As shown in FIG. 7A, the processing device 140 may connect the position of the first source and the contour of the target subject 1000 to determine tangent lines L1 and L2. Further, the processing device 140 may determine an intersection position P1 where the tangent line L1 intersects the first detector and an intersection position P2 where the tangent line L2 intersects the first detector, and designate the detector channels corresponding to the intersection positions P1 and P2 as the boundary detector channels. As shown in FIG. 7B, the processing device 140 may designate a point C1 on the detection signal curve 2000 whose horizontal coordinate corresponds to the boundary detector channel P1 and a point C2 on the detection signal curve 2000 whose horizontal coordinate corresponds to the boundary detector channel P2 as the boundary position features of the detection signal curve 2000. In some embodiments, the target subject 1000 may be approximated as an ellipse centered on the imaging center, and the two tangents may be determined based on the ellipse.

In some embodiments, the processing device may determine a position P3 on the first detector located on the side of position P1 away from position P2, and a position P4 on the first detector located on the side of position P2 away from position P1. In some embodiments, a first distance between the position P1 and position P3 and a second distance between the position P2 and position P4 may be set manually by a user (e.g., an engineer) according to an experience value or a default setting of the medical system 100. For example, the first distance or the second distance are both equal to 2 detector channels, 3 detector channels, etc. The processing device 140 may designate the detector channels corresponding to the positions P3 and P4 as the boundary detector channels. As shown in FIG. 7B, the processing device 140 may designate a point C3 on the detection signal curve 2000 whose horizontal coordinate corresponds to the boundary detector channel P3 and a point C4 on the detection signal curve 2000 whose horizontal coordinate corresponds to the boundary detector channel P4 as the boundary position features of the detection signal curve 2000.

In some embodiments, the boundary position features of the detection signal curve are determined based on intersection points between the detection signal curve and the horizontal axis.

For example, FIG. 8A is a schematic diagram illustrating an exemplary process for determining boundary position features of a detection signal curve according to some embodiments of the present disclosure. As shown in FIG. 8A, the processing device 140 may designate the intersection points C5 and C6 between the detection signal curve 2000 and the horizontal axis as the boundary position features of the detection signal curve 2000. In some embodiments, the processing device may determine a point C9 on the horizontal axis located on the side of the point C5 away from the point C6 and a point C10 on the horizontal axis located on the side of the point C6 away from the point C5. In some embodiments, a third distance between the point C9 and the point C5 and a fourth distance between the point C10 and the point C6 may be set manually by a user (e.g., an engineer) according to an experience value or a default setting of the medical system 100. For example, the third distance or the fourth distance are both equal to 2 detector channels, 3 detector channels, etc.

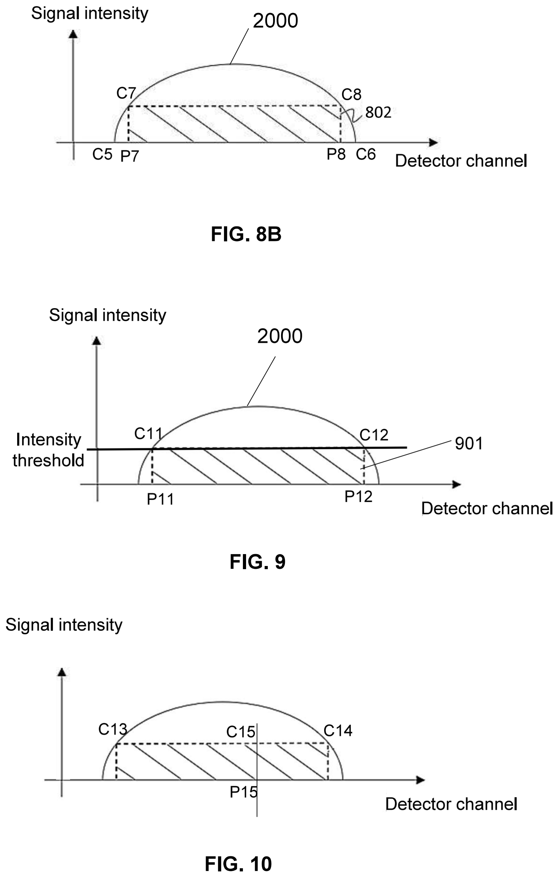

As another example, FIG. 8B is a schematic diagram illustrating an exemplary process for determining boundary position features of a detection signal curve according to some embodiments of the present disclosure. As shown in FIG. 8B, the processing device 140 may determine a point P7 on the horizontal axis located on the side of the point C5 close to the point C6 and a point P8 on the horizontal axis located on the side of the point C6 close to the point C5. In some embodiments, a fifth distance between the point P7 and the point C5 and a sixth distance between the point P8 and the point C6 may be set manually by a user (e.g., an engineer) according to an experience value or a default setting of the medical system 100. For example, the third distance or the fourth distance are both equal to 2 detector channels, 3 detector channels, etc. In some embodiments, the fifth distance or the sixth distance may be determined through a test in a similar manner as how to determine the intensity threshold. Then, the processing device 140 may designate a point C7 on the detection signal curve 2000 whose horizontal coordinate corresponds to the boundary detector channel P7 and a point C8 on the detection signal curve 2000 whose horizontal coordinate corresponds to the boundary detector channel P8 as the boundary position features of the detection signal curve 2000.

In some embodiments, the boundary position features of the detection signal curve are determined based on the detection signal curve and an intensity threshold. The intensity threshold is not greater than the maximum vertical coordinate of the detection signal curve.

For example, FIG. 9 is a schematic diagram illustrating an exemplary process for determining boundary position features of a detection signal curve according to some embodiments of the present disclosure. As shown in FIG. 9, in the coordinate system of the detection signal curve 2000, a straight line parallel to the horizontal axis and having a vertical coordinate equal to the intensity threshold may be drawn. The intersection points C11 and C12 of the straight line with the detection signal curve 2000 are determined as the boundary position features of the detection signal curve 2000. As another example, signal intensities of points in the detection signal curve may be compared with the intensity threshold. If the difference between a specific point and the intensity threshold is less than a difference threshold, the specific point (also referred to as a matching point) may be determined as a boundary position feature of the detection signal curve. In some embodiments, if there are more than two matching points, the two matching points with the minimum horizontal coordinate and the maximum horizontal coordinate may be determined as the boundary position features.

The intensity threshold may be set manually by a user (e.g., an engineer). according to an experience value or a default setting of the medical system 100. In some embodiments, the intensity threshold may be determined through a test. Merely by way of example, scan data of a reference phantom by performing a dual-source or multi-source scan on the reference phantom may be acquired. The reference phantom has a known shape, size, and well-defined scattering characteristics. The reference phantom may be made of a specific material such that its X-ray attenuation characteristics are similar to those of the target subject. Then, process 400 may be applied multiple times to the scan data of the reference phantom to obtain multiple sets of corrected data. Multiple images of the reference phantom may be reconstructed based on the multiple sets of corrected data. In each application of the correction process 400, one of a plurality of candidate intensity thresholds may be used to obtain the boundary position features, and the first convolution region may be determined based on the obtained boundary position features. Image quality parameters of the images corresponding to the candidate intensity thresholds may then be compared. Exemplary image uniformity parameters may include a contrast-to-noise ratio, a degree of suppression of cross-scattering artifacts. Finally, the candidate intensity threshold corresponding to the image with the optimal image quality may be selected as the intensity threshold. Through the above approach, the determination of the intensity threshold is directly associated with the final image quality, ensuring that the intensity threshold adopted in clinical applications can achieve optimal cross-scattering correction effects.

In some embodiments, the intensity threshold is adaptively determined based on a tube voltage used in the dual-source or multi-source scan of the target subject and/or the size of the target subject along the first direction described in FIG. 4. The first direction is perpendicular to the second direction connecting the first source of the first source-detector pair and the imaging center at the scanning angle. For example, the greater the tube voltage, the greater the intensity threshold. As another example, the greater the size of the target subject along the first direction, the greater the intensity threshold.

Experimental observations have shown that as the size of the scanned subject increases, the width (e.g., a full width at half maximum) of the detection signal curve along the detector channel direction (i.e., the horizontal axis) correspondingly increases. However, when the size of the scanned subject increases beyond a certain point, this width tends to saturate and no longer increases with further enlargement of the size of the scanned subject, indicating the existence of a maximum width threshold.

For ease of description, among the boundary position features, the one with the smaller horizontal coordinate is referred to as the starting boundary feature, and the one with the larger horizontal coordinate is referred to as the ending boundary feature. In some embodiments, if the width determined based on the starting boundary feature and the ending boundary feature (i.e., the difference between their horizontal coordinates) exceeds the maximum width threshold, an adjustment to the boundary position features may be required. In some embodiments, a new ending boundary feature may be determined based on the starting boundary feature and the maximum width threshold. For example, FIG. 10 is a schematic diagram illustrating an exemplary process for adjusting boundary position features according to some embodiments of the present disclosure. As shown in FIG. 10, the difference between horizontal coordinates of a starting boundary feature C13 and an ending boundary feature C14 exceeds the maximum width threshold. A reference horizontal coordinate P15 is obtained by adding the maximum width threshold to the horizontal coordinate of the starting boundary feature C13. Then, a straight line parallel to the vertical axis and passing through the reference horizontal coordinate P15 is drawn, and the intersection point C15 of this line with the detection signal curve is designated as the new ending boundary feature.

In 620, the processing device 140 may determine the first convolution region based on the boundary position features and the detection signal curve.

In some embodiments, when the vertical coordinates of the boundary position features are greater than 0, for each boundary position feature, the processing device 140 may determine a straight line parallel to the vertical axis and passing through the boundary position feature, and determine an intersection point of the straight line with the horizontal axis. Then, the processing device 140 may connect the boundary position features and the intersection points corresponding to the boundary position features to obtain a rectangular region, and designate the rectangular region as the first convolution region.

For example, as shown in FIG. 7B, in some embodiments, the boundary position features are point C1 and C2. For the point C1, the processing device 140 may determine a straight line parallel to the vertical axis and passing through the point C1, wherein the straight line has the intersection point P1 with the horizontal axis. For the point C2, the processing device 140 may determine a straight line parallel to the vertical axis and passing through the point C2, wherein the straight line has the intersection point P2 with the horizontal axis. Then, the processing device 140 may connect the points C1, C2, P1, and P2 to obtain a rectangular region 701, and designate the rectangular region 701 as the first convolution region. In some embodiments, the boundary position features are point C3 and C4. For the point C3, the processing device 140 may determine a straight line parallel to the vertical axis and passing through the point C3, wherein the straight line has the intersection point P3 with the horizontal axis. For the point C4, the processing device 140 may determine a straight line parallel to the vertical axis and passing through the point C4, wherein the straight line has the intersection point P4 with the horizontal axis. Then, the processing device 140 may connect the points C3, C4, P3, and P4 to obtain a rectangular region 702, and designate the rectangular region 702 as the first convolution region.

As another example, as shown in FIG. 8B, the boundary position features are point C7 and C8. For the point C7, the processing device 140 may determine a straight line parallel to the vertical axis and passing through the point C7, wherein the straight line has the intersection point P7 with the horizontal axis. For the point C8, the processing device 140 may determine a straight line parallel to the vertical axis and passing through the point C8, wherein the straight line has the intersection point P8 with the horizontal axis. Then, the processing device 140 may connect the points C7, C8, P7, and P8 to obtain a rectangular region 802, and designate the rectangular region 802 as the first convolution region.

As still another example, as shown in FIG. 9, the boundary position features are point C11 and C12. For the point C11, the processing device 140 may determine a straight line parallel to the vertical axis and passing through the point C11, and further determine an intersection point P11 of the straight line with the horizontal axis. For the point C12, the processing device 140 may determine a straight line parallel to the vertical axis and passing through the point C12, and further determine an intersection point P12 of the straight line with the horizontal axis. Then, the processing device 140 may connect the points C11, C12, P11, and P12 to obtain a rectangular region 901, and designate the rectangular region 901 as the first convolution region.

In some embodiments, when the vertical coordinates of the boundary position features are equal to 0, the processing device 140 may determine the first convolution region based on the boundary position features, the detection signal curve, and the intensity threshold. More descriptions regarding the intensity threshold may be found in operation 610. Specifically, the processing device 140 may determine a straight line parallel to the horizontal axis and having a vertical coordinate equal to the intensity threshold. Then, the processing device 140 may determine two points on the straight line whose horizontal coordinates are equal to horizontal coordinates of the boundary position features, respectively. Further, the processing device 140 may connect the two points and the boundary position features to obtain a rectangular region, and designate the rectangular region as the first convolution region.

For example, as shown in FIG. 8A, in some embodiments, the boundary position features are point C5 and C6. The processing device 140 may determine a straight line parallel to the horizontal axis and having a vertical coordinate equal to an intensity threshold I1. The processing device 140 may determine a point P5 on the straight line whose horizontal coordinate is equal to the horizontal coordinate of the point C5, and a point P6 on the straight line whose horizontal coordinate is equal to the horizontal coordinate of the point C6. Further, the processing device 140 may connect the points C5, C6, P5, and P6 to obtain a rectangular region 801, and designate the rectangular region 801 as the first convolution region.

As another example, as shown in FIG. 8A, in some embodiments, the boundary position features are point C9 and C10. The processing device 140 may determine a straight line parallel to the horizontal axis and having a vertical coordinate equal to an intensity threshold I2. The processing device 140 may determine a point P10 on the straight line whose horizontal coordinate is equal to the horizontal coordinate of the point C10, and a point P9 on the straight line whose horizontal coordinate is equal to the horizontal coordinate of the point C9. Further, the processing device 140 may connect the points C9, C10, P9, and P10 to obtain a rectangular region 803, and designate the rectangular region 803 as the first convolution region.

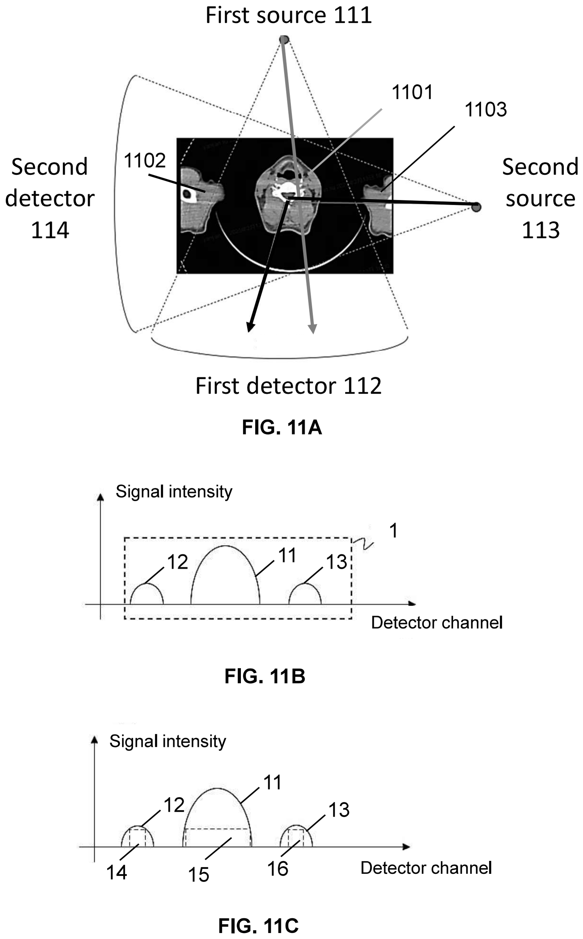

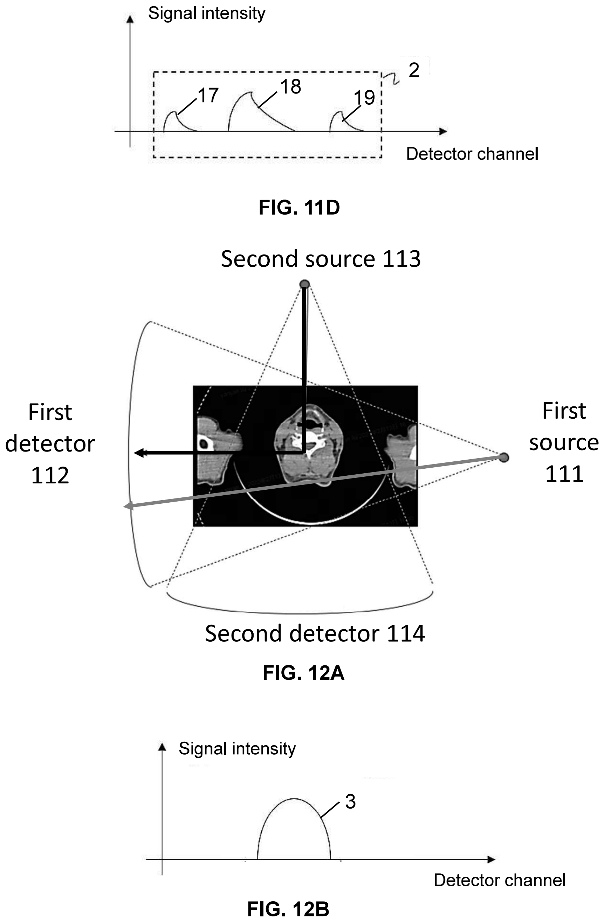

In some embodiments, the detection signal curve may include a plurality of sub-signal curves respectively corresponding to at least two scan sub-regions of the target subject. For example, FIGs. 11A-11D are schematic diagrams illustrating an exemplary a dual-source or multi-source scan on a shoulder of the target subject at a first scanning angle according to some embodiments of the present disclosure. As shown in FIG. 11A, scan sub-regions of the shoulder include the neck 1101, a first arm 1102, and a second arm 1103. At the first scanning angle, the first detector 1 is not arranged in line with the first arm 1102, the neck 1101, and the second arm 1103. As shown in FIG. 11B, a detection signal curve 1 of the first detector 111 may include a sub-signal curve 11 corresponding to the neck 1101, a sub-signal curve 12 corresponding to the first arm 1102, and a sub-signal curve 13 corresponding to the second arm 1103. As shown in FIG. 11C, a first convolution region 15 corresponding to the sub-signal curve 11, a first convolution region 14 corresponding to the sub-signal curve 12, and a first convolution region 16 corresponding to the sub-signal curve 13 may be determined. As shown in FIG. 11D, a corrected detection signal curve 2 may be determined based on the first convolution regions 14-16. The corrected detection signal curve 2 may include a corrected sub-signal curve 18 corresponding to the neck 1101, a corrected sub-signal curve 17 corresponding to the first arm 1102, and a corrected sub-signal curve 19 corresponding to the second arm 1103. The corrected sub-signal curve 18 may be determined based on the first convolution region 15, the corrected sub-signal curve 17 may be determined based on the first convolution region 14, and the corrected sub-signal curve 19 may be determined based on the first convolution region 16. In this case, by respectively determining the boundary position features of each sub-signal curve and determining the first convolution region corresponding to each sub-signal curve based on its boundary position features, a targeted analysis may be performed for each segment of the detection signal curve corresponding to different scan sub-regions, thereby improving the accuracy of identifying the first convolution regions.

For example, FIGs. 12A-12D are schematic diagrams illustrating an exemplary a dual-source or multi-source scan of a shoulder of the target subject at a second scanning angle according to some embodiments of the present disclosure. As shown in FIG. 12A, scan sub-regions of the shoulder include the neck 1101, the first arm 1102, and the second arm 1103. At the second scanning angle, the first detector 1 is arranged approximately in line with the first arm 1102, the neck 1101, and the second arm 1103. As shown in FIG. 12B, a single detection signal curve 3 is obtained. As shown in FIG. 12C, a first convolution region 31 corresponding to the detection signal curve 3 may be determined. As shown in FIG. 12D, a corrected detection signal curve 4 may be determined based on the first convolution region 31.

FIG. 13 is a flowchart illustrating an exemplary process 1300 for determining a target kernel according to some embodiments of the present disclosure. In some embodiments, the process 1300 may be performed to achieve at least part of operation 430 as described in connection with FIG. 4.

In 1310, the processing device 140 may obtain primary signal intensities detected by the plurality of detector channels during a first scan of a target phantom, wherein a first source of the first source-detector pair is activated and the second source is disactivated during the first scan.

The target phantom may have attenuation characteristics similar to those of the target subject to ensure that the target kernel can accurately reflect the cross-scattering characteristics of the target subject. In some embodiments, the target phantom may have material compositions similar to those of the target subject. For instance, when the target subject is a human patient, water phantoms are commonly employed as suitable candidate phantoms due to the fact that the X-ray attenuation properties of water closely approximate those of human soft tissue.

In some embodiments, the phantom size of the target phantom is similar to a size of the target subject along a first direction, the first direction is perpendicular to a second direction connecting a first source of the first source-detector pair and an imaging center at the scanning angle. More descriptions regarding the first direction and the second direction may be found elsewhere in the present disclosure (e.g., operation 430 in FIG. 4 and the descriptions thereof).

The signal intensities detected by the detector channels of the first detector during the first scan include only primary signal intensities and are free of cross-scattering signal intensities, since during the first scan, the first source of the first source-detector pair is activated while the second source is deactivated.

In some embodiments, the processing device 140 may obtain the primary signal intensities from one or more components of the medical system 100 (e.g., the storage device 150, the terminals(s) 130) or an external source via a network (e.g., the network 120). For example, the primary signal intensities may be stored in the storage device 150. The processing device 140 may access the storage device 150 and retrieve the primary signal intensities.

In 1320, the processing device 140 may obtain cross-scattering signal intensities detected by the plurality of detector channels during a second scan of the target phantom, wherein the first source is disactivated and the second source is activated during the second scan.

The signal intensities detected by the detector channels of the first detector during the second scan include only cross-scattering signal intensities and are free of primary signal intensities, since during the second scan, the first source is deactivated while the second source is activated.

In some embodiments, the processing device 140 may obtain the cross-scattering signal intensities in a similar manner how to obtain the primary signal intensities.

In 1330, the processing device 140 may determine the target kernel based on the primary signal intensities and the cross-scattering signal intensities.

In some embodiments, the processing device 140 may determine a second convolution region based on a second detection signal curve corresponding to the primary signal intensities. In some embodiments, the second detection signal curve may be generated in a similar manner how to generate the detection signal curve described in operation 410. In some embodiments, the second convolution region may be generated in a similar manner how to generate the first convolution region described in FIG. 6 is generated. Further, the processing device 140 may determine the target kernel by performing a deconvolution operation on the cross-scattering signal intensities based on the second convolution region. The deconvolution operation refers to the inverse operation of a convolution operation.

By determining the second convolution region using the same approach as for the first convolution region, it improves the adaptability between the target kernel derived from the deconvolution process and the actual correction process involving the first convolution region, thereby enhancing the accuracy of the estimated cross-scattering information.

To suppress cross-scattering, a scatter suppression component such as an anti-scatter grid (ASG) is typically installed for each detector in the dual-source or multi-source CT device. However, since the scatter suppression component includes scatter suppression members (e.g., blades of the ASG) corresponding to different detector channels, the installation precision (such as slight deviations in installation angle or position) varies among the scatter suppression members, leading to inconsistent scatter suppression efficiencies across different detector channels. This inconsistency introduces additional high-frequency components into the cross-scattering signals. When performing cross-scattering correction, if the high-frequency component is not taken into account, it may lead to the estimated cross-scattering signal intensities deviating from their actual cross-scattering signal intensities. For instance, the estimated cross-scattering signal intensities calculated in the 430 mainly corresponds to the low-frequency cross-scattering signal, without considering the high-frequency cross-scattering signals. This results in the corrected signal intensities still containing noise caused by the high-frequency cross-scattering signals. Therefore, even if the cross-scattering correction methods are applied to correct original signal intensities detected by the dual-source or multi-source CT device, artifacts (e.g., ring or band artifacts) caused by the high-frequency cross-scattering signals may still appear in an image reconstructed from the corrected signals, affecting image quality.

To address the above issues, the present disclosure provides a cross-scattering correction method, to effectively eliminate or reduce the interference caused by variations in the installation of scatter suppression members and inconsistent scatter suppression efficiencies across different detector channels, and reduce the impact of the cross-scattering signals in the high-frequency component.

FIG. 15 is a flowchart illustrating an exemplary cross-scattering correction process 1500 for a dual-source or multi-source scan according to some embodiments of the present disclosure. The dual-source or multi-source scan may be performed by a dual-source or multi-source CT device including a plurality of detectors. The plurality of detectors include a first detector having a plurality of detector channels. For each of the plurality of detector channels, process 1500 may be performed to determine a corrected signal intensity of the detector channel.

In 1510, the processing device 140 may determine an estimated cross-scattering signal intensity of a detector channel of the first detector based on signal intensities detected by the plurality of detector channels at a scanning angle during the dual-source or multi-source scan of a target subject.

In some embodiments, the processing device 140 may determine the estimated cross-scattering signal intensity of the detector channel using a similar convolution-based approach as described in operations 410-430.

In 1520, the processing device 140 may determine a signal intensity error of the detector channel based on the estimated cross-scattering signal intensity of the detector channel and a mapping relationship between cross-scattering signal intensities and signal intensity errors corresponding to the detector channel.

The signal intensity error (also referred to as the signal intensity jump amplitude) may be a quantitative value used to quantify the degree of enhancement or attenuation of the cross-scattering signal intensity of the detector channel caused by inconsistent scatter suppression efficiency across different detector channels. The cross-scattering signal intensity detected by a particular detector channel undergoes significant changes (either an increase or a decrease) relative to other detector channels, resulting in significant changes in the detected signal intensity of the detector channel. The signal intensity error may be regarded as an intensity of the cross-scattering signals in the high frequency.

As mentioned above, the installation precision varies among the scatter suppression members of the scatter suppression component, leading to inconsistent scatter suppression efficiency across different detector channels. This inconsistency causes the cross-scattering signal intensities actually received by some detector channels exhibits enhancement or attenuation (e.g., undergo discontinuous changes relative to other detector channels). The mapping relationship (also referred to as signal intensity jump relationship) corresponding to a detector channel provides a quantitative description of how the inconsistency in scatter suppression efficiency affects the detector channel. For example, the mapping relationship may be used to describe the signal intensity errors (i.e., an intensity of high-frequency cross-scattering signals) introduced by differences in scatter suppression efficiency under different cross-scattering signal intensities.

In some embodiments, each detector channel has its own independent mapping relationship. This is because the physical positions of different detector channels and the installation status of their corresponding scatter suppression members (e.g., ASG blades) may be different, thus requiring a mapping relationship to be established separately for each detector channel.

In some embodiments, the mapping relationship may be expressed in a functional form. For example, the mapping relationship may be expressed in the following Equation (2): ${Y}_{error} = f \left(X\right) ,$ (2) where, *Yₑᵣᵣₒᵣ* denotes the signal intensity error, *f*(*X*) denotes a function, *X* denotes the cross-scattering signal intensity.

In some embodiments, the mapping relationship may be represented as a linear relationship. For example, the mapping relationship may be expressed in the following Equation (3): ${Y}_{error} = kx + b ,$ (3) where, *Yₑᵣᵣₒᵣ* denotes the signal intensity error, *x* denotes the cross-scattering signal intensity, and *k* and b denote coefficients.

In some embodiments, the mapping relationship may be recorded in the form of a curve. Specifically, the mapping relationship may be generated with cross-scattering signal intensities along a horizontal axis and signal intensity errors along a vertical axis.

In some embodiments, the processing device 140 may obtain the mapping relationship from one or more components of the medical system 100 (e.g., the storage device 150, the terminals(s) 130) or an external source via a network (e.g., the network 120). For example, the mapping relationship may be generated previously by a computing device (e.g., the processing device 140), and stored in the storage device 150. The processing device 140 may access the storage device 150 and retrieve the mapping relationship.

In some embodiments, the mapping relationship corresponding to the detector channel is determined based on a plurality of sets of reference cross-scattering signal intensities detected by the first detector in a plurality of reference scans. The plurality of reference scans may be performed by the dual-source or multi-source CT device. In each reference scan, each detector channel of the first detector collects a corresponding cross-scattering signal intensity. Each set of reference cross-scattering signal intensities may be detected by the detector channels of the first detector in one reference scan.

The plurality of reference scans are performed on a plurality of phantoms with different sizes or on one phantom at different positions. The phantoms may have attenuation characteristics similar to those of the target subject. In some embodiments, the phantoms may have material compositions similar to those of the target subject. For instance, when the target subject is a human patient, water phantoms are commonly employed as suitable candidate phantoms due to the fact that the X-ray attenuation properties of water closely approximate those of human soft tissue. In some embodiments, the position of phantom may be the position of the phantom relative to the imaging center of the dual-source or multi-source CT device.

The first source of the first source-detector pair is disactivated and the second source is activated during the plurality of reference scans. In such case, the signal intensities detected by the first detector during the reference scans include only cross-scattering signal intensities, since the first source is deactivated while the second source is activated.

By using phantoms with different sizes or changing the position of the same phantom, the cross-scattering signal intensities collected by the same detector channel in the reference scans may be different. In some embodiments, when performing the reference scans on the phantoms of different sizes, the phantoms of various sizes may be placed at the same position (e.g., the imaging center) and scanned separately. When performing reference scans on one phantom at different positions, the same phantom may be placed at different positions and scanned separately.

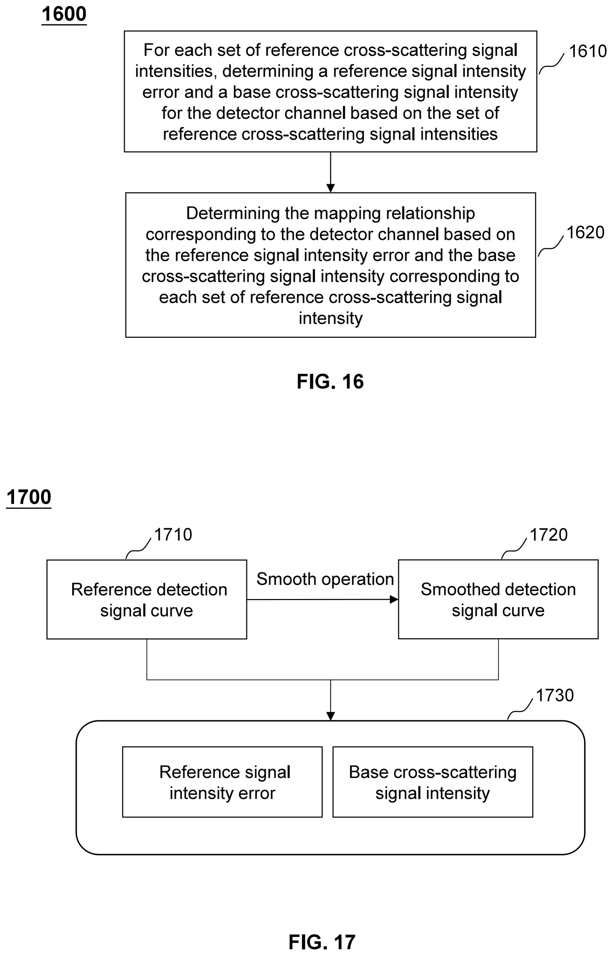

In some embodiments, the mapping relationship corresponding to the detector channel may be determined by performing the following operations. Specifically, for each set of reference cross-scattering signal intensities, the processing device 140 may determine a reference signal intensity error and a base cross-scattering signal intensity for the detector channel based on the set of reference cross-scattering signal intensities. Further, the processing device 140 may determine the mapping relationship corresponding to the detector channel based on the reference signal intensity error and the base cross-scattering signal intensity corresponding to each set of reference cross-scattering signal intensity. More descriptions regarding the determination of the mapping relationship may be found elsewhere in the present disclosure (e.g., FIG. 16 and the descriptions thereof).

In some embodiments, the first detector comprises a plurality of detector modules. Within each detector module, the detector channels share a common mapping relationship. Specifically, in the dual-source or multi-source CT device, the first detector typically includes multiple detector modules, each including several detector channels. During manufacturing and installation processes, the scatter suppression members (e.g., the blades of ASG) corresponding to multiple channels within the same detector module often have relatively close installation precision, and therefore the scatter suppression efficiencies of these detector channels are also relatively similar. Based on this characteristic, the detector channels may be grouped according to the detector module to which they belong, such that all detector channels within the same detector module share the same mapping relationship.

In some embodiments, for each set of reference cross-scattering signal intensities, a reference signal intensity error and a base cross-scattering signal intensity corresponding to each detector channel may be determined respectively. Then, for each detector module, reference signal intensity errors of the detector channels of the detector module may be averaged to obtain an average reference intensity error for the detector module, and the base cross-scattering signal intensities of the detector channels of the detector module may be averaged to obtain an average base cross-scattering signal intensity for the detector module. Further, for each detector module, the mapping relationship corresponding to the detector module may be determined based on the average reference intensity error and the average base cross-scattering signal intensity corresponding to each reference scan.

In this way, by averaging data from multiple detector channels within the same detector module, correction distortions caused by noise or random fluctuations in a single channel can be effectively avoided or reduced, thereby improving the robustness and signal-to-noise ratio of the cross-scatter estimation. Moreover, since the hardware differences between different detector modules are typically greater than the differences among detector channels within the same detector module, the approach of sharing a common mapping relationship within the same detector module is sufficient to accurately characterize its scattering behavior. This significantly reduces the number of parameters requiring calibration while ensuring correction accuracy, thereby lowering the complexity and computational cost of system calibration. In addition, the above grouping process makes the cross-scattering correction more efficient and stable in engineering implementation, which is beneficial for improving the overall performance and reliability of the system.

In a dual-source or multi-source CT scan, the tube voltage is an important factor affecting cross-scattering signal intensities. Different tube voltages lead to changes in the energy spectrum distribution of X-rays, which in turn affects the number of scattered photons generated, their energy distribution, and penetration characteristics. This results in different patterns in the cross-scattering signal intensities received by the detector channels and the corresponding signal intensity errors caused by the scatter suppression component. To more accurately characterize scattering behavior under different scanning conditions, in some embodiments, the present disclosure provides a mapping relationship construction scheme based on tube voltage.

Specifically, for each detector channel, the processing device 140 may determine the mapping relationship corresponding to the detector channel from a plurality of candidate mapping relationships corresponding to the detector channel based on a tube voltage used in the dual-source or multi-source scan. The plurality of candidate mapping relationships are predetermined based on different tube voltages. In this case, the above reference scans need to be performed not only under phantom conditions of different sizes or different positions, but also under multiple different tube voltage conditions. For example, for each phantom condition (specific size or specific position), reference scans may be performed at multiple different tube voltages, thereby acquiring multiple sets of reference cross-scattering signal intensities. Then, the process 1600 illustrated in FIG. 16 may be employed to determine the candidate mapping relationships.

By introducing the tube voltage dimension, this embodiment can further refine the construction process of the mapping relationship, making the determined signal intensity error more consistent with the physical laws under actual scanning conditions, and can significantly improve the accuracy and adaptability of cross-scattering correction.

In 1530, the processing device 140 may determine a corrected cross-scattering signal intensity of the detector channel based on the signal intensity error and the estimated cross-scattering signal intensity of the detector channel.

In some embodiments, the processing device 140 may determine the corrected cross-scattering signal intensity of the detector channel by adjusting the estimated cross-scattering signal intensity based on the signal intensity error. For example, the processing device 140 may determine the corrected cross-scattering signal intensity of the detector channel by adding the signal intensity error into the estimated cross-scattering signal intensity. In this way, the corrected cross-scattering signal intensity is the intensity of the cross-scattering signals in both the high frequency and the low frequency.

In 1540, the processing device 140 may determine a corrected signal intensity of the detector channel by performing the cross-scattering correction on the signal intensity of the detector channel based on the corrected cross-scattering signal intensity of the detector channel.

In some embodiments, the operation 1540 may be performed in the same or similar manner as operation 440, except the estimated cross-scattering signal intensity needs to be replaced by the corrected cross-scattering signal intensity.

According to process 1500, the mapping relationship corresponding to the detector channel can quantify the signal intensity error introduced by differences in scatter suppression efficiency under different cross-scattering signal intensities. When correcting actual scan data, the estimated cross-scattering signal intensity may be corrected based on the signal intensity error determined using the mapping relationship to obtain the accurate corrected cross-scattering signal intensity, and the corrected signal intensity of the detector channel can be obtained based on the corrected cross-scattering signal intensity. Since the corrected cross-scattering signal intensity is the intensity of the cross-scattering signals in both the high frequency and the low frequency, high-frequency noises superimposed due to differences in scatter suppression efficiency may be also corrected in the cross-scattering correction, enabling the corrected signal intensity of the detector channel be more accurate. This way significantly improves the accuracy of cross-scattering correction and effectively suppresses artifacts (e.g., ring or band artifacts) in the reconstructed image.

FIG. 16 is a flowchart illustrating an exemplary process 1600 for determining a mapping relationship according to some embodiments of the present disclosure.

In some embodiments, the process 1600 may be performed to achieve at least part of operation 1520 as described in connection with FIG. 15.

In 1610, the processing device 140 may, for each set of reference cross-scattering signal intensities, determine a reference signal intensity error and a base cross-scattering signal intensity for the detector channel based on the set of reference cross-scattering signal intensities.

The reference signal intensity error refers to an estimated signal intensity error (i.e., an intensity of high-frequency cross-scattering signal) of the detector channel caused by the differences in scatter suppression efficiency.

The base cross-scattering signal intensity refers to a cross-scattering signal intensity that the detector channel should theoretically detect under ideal conditions (i.e., assuming no difference in scatter suppression efficiency, or that the scatter suppression efficiency is uniform). The base cross-scattering signal intensity can be understood as the cross-scattering signal intensity in the low frequency.

For example, for the detector channel, if a significant increase occurs from signal strength A to signal strength B, signal strength A may be referred to as the base cross-scattering signal intensity, and the change value between signal strength B and signal strength A may be referred to as the reference signal intensity error.

In some embodiments, the processing device 140 may, for each set of reference cross-scattering signal intensities, determine the reference signal intensity error and the base cross-scattering signal intensity for the detector channel based on a reference detection signal curve corresponding to the set of reference cross-scattering signal intensities. Specifically, FIG. 17 is a schematic diagram illustrating an exemplary process 1700 for determining a reference signal intensity error and a base cross-scattering signal intensity of a detector channel according to some embodiments of the present disclosure.

In 1710, the processing device 140 may obtain a reference detection signal curve corresponding to the set of reference cross-scattering signal intensities. The reference detection signal curve reflects, as a function of detector channels, the distribution of the reference cross-scattering signal intensities detected by the detector channels of the first detector during the reference scan for generating the set of reference cross-scattering signal intensities. In some embodiments, the reference detection signal curve may be generated in a similar manner as how to generate the detection signal curve. For example, the reference detection signal curve may be generated with detector channels along a horizontal axis and reference cross-scattering signal intensities along a vertical axis.

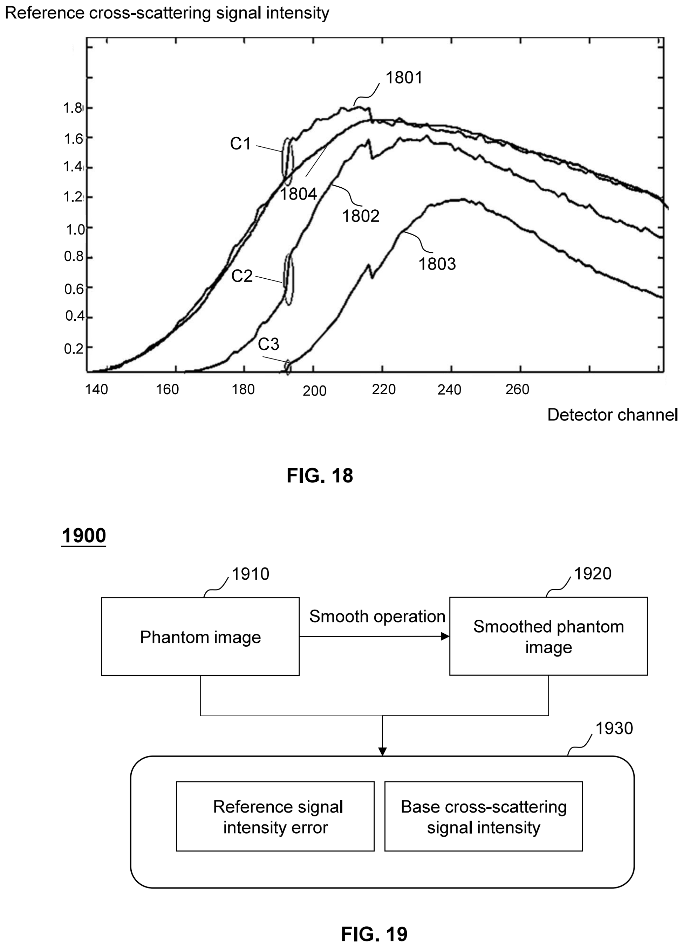

For example, FIG. 18 is a schematic diagram illustrating exemplary reference detection signal curves according to some embodiments of the present disclosure. As shown in FIG. 18, three reference scans are performed by the dual-source or multi-source CT device, and reference detection signal curves 1801-1803 corresponding to the three reference scans are obtained. The reference detection signal curves 1801-1803 exhibit several protrusions and depressions, the positions of which correspond to channel regions where relatively significant changes in signal intensity occur. For example, around the detector channel 190, at positions of a region C1 on the reference detection signal curve 1801, a region C2 on the reference detection signal curve 1802, and a region C3 on the reference detection signal curve 1803, which are circled in the figure, relatively significant changes in signal intensity occur. The base cross-scattering signal intensities corresponding to the three regions C1-C3 are around 0, 0.5, and 1.3, respectively.

In 1720, the processing device 140 may determine a smoothed detection signal curve by performing a smooth operation on the reference detection signal curve. The smoothed detection signal curve can reflect the trend of cross-scattering signal intensity distribution under ideal conditions (i.e., assuming uniform scatter suppression efficiency across the detector channels). As shown in FIG. 18, the processing device 140 may determine a smoothed detection signal curve 1804 by performing a smooth operation on the reference detection signal curve 1801. In some embodiments, the smooth operation may be performed using various signal curve smoothing algorithms, such as a moving average algorithm, a Gaussian filtering algorithm, a median filtering algorithm, a wavelet transform algorithm, etc., and the present disclosure is not limited in this regard.

In 1730, the processing device 140 may determine the reference signal intensity error and the base cross-scattering signal intensity of the detector channel based on the reference detection signal curve and the smoothed detection signal curve.

In some embodiments, the processing device 140 may determine a signal intensity difference of the detector channel based on a difference between the reference detection signal curve and the smoothed detection signal curve. Specifically, the processing device 140 may determine a first signal intensity on the reference detection signal curve corresponding to the detector channel and a second signal intensity on the smoothed detection signal curve corresponding to the detector channel. The processing device 140 may designate the second signal intensity on the smoothed detection signal curve as the base cross-scattering signal intensity of the detector channel. Then, the processing device 140 may determine the difference between the first signal intensity and the second signal intensity as the signal intensity difference of the detector channel.

The processing device 140 may determine whether the signal intensity difference satisfies a preset condition. For example, the preset condition is that the signal intensity difference is greater than an intensity difference threshold. Specifically, if the signal intensity difference is greater than the intensity difference threshold, the processing device 140 may determine that the signal intensity difference satisfies the preset condition. If the signal intensity difference is not greater than the intensity difference threshold, the processing device 140 may determine that the signal intensity difference does not satisfy the preset condition. The intensity difference threshold may be set manually by a user (e.g., an engineer) according to an experience value or a default setting of the medical system 100.

In response to determining that the signal intensity difference satisfies the preset condition, the processing device 140 may designate the signal intensity difference as the reference signal intensity error of the detector channel. In response to determining that the signal intensity difference does not satisfy the preset condition, the processing device 140 may set the reference signal intensity error of the detector channel to 0. This indicates that no significant change in signal intensity has occurred for the detector channel compared with surrounding detector channels, and therefore no additional correction of the cross-scattering signal intensity for detector channel is required.

In some embodiments, the processing device 140 may select a neighboring detector channel whose reference signal intensity error is equal to 0, and designate the reference cross-scattering signal intensity of the neighboring detector channel as the base cross-scattering signal intensity of the detector channel.

According to some embodiments of the present disclosure, the reference signal intensity error and the base cross-scattering signal intensity for the detector channel can be efficiently extracted based on the reference detection signal curve and the smoothed detection signal curve without the need for complex physical modeling or iterative calculations, thereby significantly improving the efficiency and simplicity of obtaining the mapping relationship.

In some embodiments, the processing device 140 may, for each set of reference cross-scattering signal intensities, determine the reference signal intensity error and the base cross-scattering signal intensity for the detector channel based on a phantom image corresponding to the set of reference cross-scattering signal intensities. Specifically, FIG. 19 is a schematic diagram illustrating an exemplary process 1900 for determining a reference signal intensity error and a base cross-scattering signal intensity of a detector channel according to some embodiments of the present disclosure.

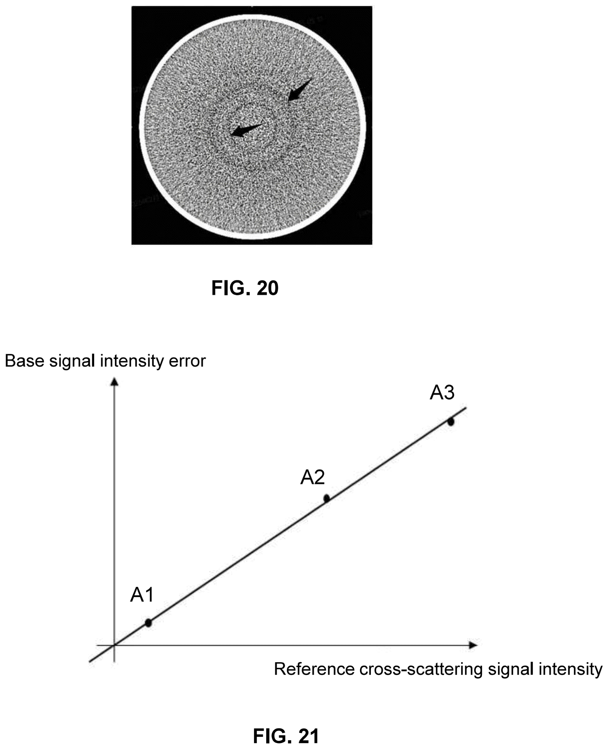

In 1910, the processing device 140 may generate a phantom image based on the set of reference cross-scattering signal intensity. In some embodiments, the processing device 140 may generate the phantom image based on the set of reference cross-scattering signal intensity using an image reconstruction algorithm. Exemplary image reconstruction algorithms include a filtered back projection (FBP) algorithm, an iterative reconstruction algorithm, a deep learning-based reconstruction algorithm, or the like. As described elsewhere in the present disclosure, inconsistent scatter suppression efficiency across different detector channels introduces additional high-frequency components into the cross-scattering signals, resulting in artifacts (e.g., ring or band artifacts) in the phantom image. For example, FIG. 20 is a schematic diagram illustrating an exemplary process phantom image according to some embodiments of the present disclosure. As shown in FIG. 20, the phantom image may include ring-shaped or band-shaped artifact regions indicated by arrows.

In 1920, the processing device 140 may generate a smoothed phantom image by performing a smooth operation on the phantom image. The smoothed phantom image may represent a phantom image obtained under ideal conditions (i.e., assuming uniform scatter suppression efficiency across the detector channels). In some embodiments, the smoothing operation may be performed on artifact regions exhibiting abnormal pixel values (e.g., the ring-shaped artifact regions shown in FIG. 20). In some embodiments, the smoothing operation may be performed on the entire phantom image. The processing device 140 may generate the smoothed phantom image using an image smoothing algorithm. Exemplary image smoothing algorithms include a mean filtering algorithm, a Gaussian filtering algorithm, a median filtering algorithm, a bilateral filtering algorithm, or the like.

In 1930, the processing device 140 may determine the reference signal intensity error and the base cross-scattering signal intensity of the detector channel based on the phantom image and the smoothed phantom image. For example, the processing device 140 may determine the reference signal intensity error and the base cross-scattering signal intensity of the detector channel based on a difference between the phantom image and the smoothed phantom image

In some embodiments, the processing device 140 may determine a difference image between the phantom image and the smoothed phantom image. The difference image contains image information related to the artifacts in the phantom image. Specifically, the processing device 140 may perform a pixel-by-pixel subtraction operation on the phantom image and the smoothed phantom image. For each pixel in the phantom image, the processing device 140 identifies a corresponding pixel in the smoothed phantom image, and subtracts the pixel value of the corresponding pixel in the smoothed phantom image from the pixel value of the pixel in the phantom image to obtain a pixel value difference for each pixel. Then, the processing device 140 may generate the difference image based on the pixel value differences for each pixel.

Further, the processing device 140 may obtain forward projection data of the difference image. Specifically, the processing device 140 may perform a forward projection operation on the difference image to obtain the forward projection data. The forward projection operation may simulate signal acquisition process in a reference scan. The processing device 140 may convert the pixel value differences in an image domain into signal intensity differences in a projection domain. That is, the forward projection data includes a signal intensity difference corresponding to each detector channel. In some embodiments, the forward projection intensity corresponding to each detector channel in the forward projection data may be directly designated as its signal intensity difference. Alternatively, the signal intensity difference of a detector channel may be determined based on the forward projection intensity of the detector channel in the forward projection data and a positive correlation between the forward projection intensity and the signal intensity difference.

Then, the processing device 140 may determine the reference signal intensity error and the base cross-scattering signal intensity of the detector channel based on the forward projection data.

In some embodiments, the processing device 140 may determine whether the signal intensity difference of the detector channel satisfies the preset condition.

In response to determining that the signal intensity difference satisfies the preset condition, the processing device 140 may designate the signal intensity difference as the reference signal intensity error of the detector channel. In response to determining that the signal intensity difference does not satisfy the preset condition, the processing device 140 may set the reference signal intensity error of the detector channel to 0.

In some embodiments, the processing device 140 may determine an error detection signal curve based on the forward projection data. The error detection signal curve may be generated in a similar manner as how to generate the reference detection signal curve. Further, the processing device 140 may determine the base cross-scattering signal intensity of the detector channel based on the reference detection signal curve and the error detection signal curve. Specifically, the processing device 140 may determine the first signal intensity on the reference detection signal curve corresponding to the detector channel and a third signal intensity on the error detection signal curve corresponding to the detector channel. The processing device 140 may determine a difference between the first signal intensity and the third signal intensity, and designate the difference as the base cross-scattering signal intensity of the detector channel.

In some embodiments, the processing device 140 may select a neighboring detector channel whose reference signal intensity error is equal to 0, and designate the reference cross-scattering signal intensity of the neighboring detector channel as the base cross-scattering signal intensity of the detector channel.

According to some embodiments of the present disclosure, the reference signal intensity error and the base cross-scattering signal intensity for a detector channel may be determined based on the phantom image and the smoothed phantom image. This image processing-based approach leverages spatial information in the image domain more comprehensively, enabling accurate determination of these parameters and thereby significantly improving the accuracy of the resulting mapping relationship. As this method involves computational processes such as image reconstruction and forward projection, it may be particularly suitable for scenarios where computing resources are sufficient (e.g., the amount of computing resources satisfies a preset condition) or where high accuracy is required for detecting significant changes in signal intensity.

In 1620, the processing device 140 may determine the mapping relationship corresponding to the detector channel based on the reference signal intensity error and the base cross-scattering signal intensity corresponding to each set of reference cross-scattering signal intensity.

For the detector channel, multiple data points may be obtained. Each data point consists of the reference signal intensity error and the base cross-scattering signal intensity corresponding to one reference scan (i.e., one set of reference cross-scattering signal intensity).

In some embodiments, the processing device 140 may perform functional fitting based on the data points to establish the mapping relationship corresponding to the detector channel. The fitting may employ various suitable functional forms, such as a linear function (e.g., Equation (3)), a polynomial function, an exponential function, or other nonlinear functions. In some embodiments, the mapping relationship may also be stored in the form of a look-up table. Fitting methods may include the least squares method, interpolation methods, etc., and the present application is not limited in this regard.

For example, FIG. 21 is a schematic diagram illustrating an exemplary mapping relationship according to some embodiments of the present disclosure. As shown in FIG. 21, three reference scans are performed, three data points A1- A3 may be determined for a detector channel. The processing device 140 may determine the mapping relationship corresponding to the detector channel by fitting the points A1-A3 using a linear function.

In some cases, for certain detector channels, if their corresponding reference signal intensity errors are zero (or are all below a preset error threshold) in all reference scans, indicating that the scatter suppression efficiency for the detector channels are relatively ideal and no significant signal intensity change occurs, the mapping relationships for the detector channels may be directly set as a zero function (i.e., the signal intensity error is constantly zero). This means that the estimated cross-scattering signal intensities for the detector channels do not require correction.

It should be noted that the processes 400, 600, 1300, 1500, and 1600 and the descriptions thereof are provided for the purposes of illustration, and not intended to limit the scope of the present disclosure. For persons having ordinary skills in the art, various modifications and changes in the forms and details of the application of the above method and system may occur without departing from the principles of the present disclosure. However, those variations and modifications also fall within the scope of the present disclosure. For example, the operations of the illustrated processes 400, 600, 1300, 1500, and 1600 are intended to be illustrative. In some embodiments, the processes 400, 600, 1300, 1500, and 1600 may be accomplished with one or more additional operations not described, and/or without one or more of the operations discussed. Additionally, the order in which the operations of the processes 400, 600, 1300, 1500, and 1600 and regarding descriptions are not intended to be limiting.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of this disclosure.

Moreover, certain terminology has been used to describe embodiments of the present disclosure. For example, the terms "one embodiment," "an embodiment," and "some embodiments" mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

A computer readable signal medium may include a propagated data signal with computer readable program code embodied therein, for example, in baseband or as part of a carrier wave. Such a propagated signal may take any of a variety of forms, including electro-magnetic, optical, or the like, or any suitable combination thereof. A computer readable signal medium may be any computer readable medium that is not a computer readable storage medium and that may communicate, propagate, or transport a program for use by or in connection with an instruction execution system, apparatus, or device. Program code embodied on a computer readable signal medium may be transmitted using any appropriate medium, including wireless, wireline, optical fiber cable, RF, or the like, or any suitable combination of the foregoing.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claim subject matter lies in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate a certain variation (e.g., ±1%, ±5%, ±10%, or ±20%) of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable. In some embodiments, a classification condition used in classification or determination is provided for illustration purposes and modified according to different situations. For example, a classification condition that "a value is greater than the threshold value" may further include or exclude a condition that "the probability value is equal to the threshold value."

## Claims

1. A method for cross-scattering correction for a dual-source or multi-source computed tomography (CT) device, wherein the dual-source or multi-source CT device at least includes a first source-detector pair and a second source-detector pair, the method is implemented on a computing device having at least one processor and at least one storage device, and the method comprises:
obtaining a detection signal curve of a first detector of the first source-detector pair, wherein the detection signal curve is generated based on signal intensities detected by a plurality of detector channels of the first detector at a scanning angle during a dual-source or multi-source scan of a target subject;
determining a first convolution region based on the detection signal curve;
determining estimated cross-scattering signal intensities of the plurality of detector channels by performing a convolution operation on the first convolution region using a target kernel, wherein the target kernel characterizes scattering contributions from a second source of the second source-detector pair to the first detector; and
determining corrected signal intensities of the plurality of detector channels by performing a cross-scattering correction on the signal intensities of the plurality of detector channels based on the estimated cross-scattering signal intensities of the plurality of detector channels.

2. The method of claim 1, wherein the determining a first convolution region based on the detection signal curve comprises:
determining boundary position features of the detection signal curve, wherein the boundary position features indicate projection boundaries of the target subject on the first detector at the scanning angle; and
determining the first convolution region based on the boundary position features and the detection signal curve.

3. The method of claim 1 or 2, wherein the target kernel is determined by:
obtaining primary signal intensities detected by the plurality of detector channels during a first scan of a target phantom, wherein a first source of the first source-detector pair is activated and the second source is disactivated during the first scan;
obtaining cross-scattering signal intensities detected by the plurality of detector channels during a second scan of the target phantom, wherein the first source is disactivated and the second source is activated during the second scan; and
determining the target kernel based on the primary signal intensities and the cross-scattering signal intensities.

4. The method of claim 3, wherein the determining the target kernel based on the primary signal intensities and the cross-scattering signal intensities comprises:
determining a second convolution region based on a detection signal curve corresponding to the primary signal intensities; and
determining the target kernel by performing a deconvolution operation on the cross-scattering signal intensities based on the second convolution region.

5. The method of any one of claims 1-4, wherein the method further comprises:
determining a size of the target subject along a first direction, wherein the first direction is perpendicular to a second direction connecting a first source of the first source-detector pair and an imaging center at the scanning angle; and
determining the target kernel from a plurality of candidate kernels based on the size of the target subject, wherein the plurality of candidate kernels are predetermined based on candidate phantoms having different phantom sizes.

6. The method of any one of claims 1-5, wherein the method further comprises:
determining whether a termination condition is satisfied based on the estimated cross-scattering signal intensities and the signal intensities;
in response to determining that the termination condition is not satisfied, designating the corrected signal intensities as the signal intensities and repeating the method of claim 1 until the termination condition is satisfied.

7. The method of any one of claims 1-6, wherein the determining the corrected signal intensities of the plurality of detector channels comprises:
for each of the plurality of detector channels,
determining a signal intensity error of the detector channel based on the estimated cross-scattering signal intensity of the detector channel and a mapping relationship between cross-scattering signal intensities and signal intensity errors corresponding to the detector channel;
determining a corrected cross-scattering signal intensity of the detector channel based on the signal intensity error and the estimated cross-scattering signal intensity of the detector channel;
determining the corrected signal intensity of the detector channel by performing the cross-scattering correction on the signal intensity of the detector channel based on the corrected cross-scattering signal intensity of the detector channel.

8. The method of claim 7, wherein the mapping relationship corresponding to the detector channel is determined based on a plurality of sets of reference cross-scattering signal intensities detected by the first detector in a plurality of reference scans,
the plurality of reference scans are performed on a plurality of phantoms with different sizes or on one phantom at different positions, and
a first source of the first source-detector pair is disactivated and the second source is activated during the plurality of reference scans.

9. The method of claim 8, wherein the mapping relationship corresponding to the detector channel is determined by:
for each set of reference cross-scattering signal intensities, determining a reference signal intensity error and a base cross-scattering signal intensity for the detector channel based on the set of reference cross-scattering signal intensities; and
determining the mapping relationship corresponding to the detector channel based on the reference signal intensity error and the base cross-scattering signal intensity corresponding to each set of reference cross-scattering signal intensity.

10. The method of claim 9, wherein the determining a reference signal intensity error and a base cross-scattering signal intensity for the detector channel based on the set of reference cross-scattering signal intensities further comprises:
obtaining a reference detection signal curve corresponding to the set of reference cross-scattering signal intensities;
determining a smoothed detection signal curve by performing a smooth operation on the reference detection signal curve; and
determining the reference signal intensity error and the base cross-scattering signal intensity of the detector channel based on the reference detection signal curve and the smoothed detection signal curve.

11. The method of claim 9, wherein the determining a reference signal intensity error and a base cross-scattering signal intensity for the detector channel based on the set of reference cross-scattering signal intensities further comprises:
generating a phantom image based on the set of reference cross-scattering signal intensity;
generating a smoothed phantom image by performing a smooth operation on the phantom image; and
determining the reference signal intensity error and the base cross-scattering signal intensity of the detector channel based on the phantom image and the smoothed phantom image.

12. The method of any one of claims 7-11, wherein the first detector comprises a plurality of detector modules, and
within each detector module, the detector channels share a common mapping relationship.

13. The method of any one of claims 7-12, wherein the method further comprises:
for each detector channel, determining the mapping relationship corresponding to the detector channel from a plurality of candidate mapping relationships corresponding to the detector channel based on a tube voltage used in the dual-source or multi-source scan, wherein the plurality of candidate mapping relationships are predetermined based on different tube voltages.

14. A system, comprising:
at least one storage device storing a set of instructions for cross-scattering correction for a dual-source or multi-source computed tomography (CT) device, wherein the dual-source or multi-source CT device at least includes a first source-detector pair and a second source-detector pair; and
at least one processor configured to communicate with the at least one storage device, wherein when executing the set of instructions, the at least one processor is configured to direct the system to perform operations including:
obtaining a detection signal curve of a first detector of the first source-detector pair, wherein the detection signal curve is generated based on signal intensities detected by a plurality of detector channels of the first detector at a scanning angle during a dual-source or multi-source scan of a target subject;
determining a first convolution region based on the detection signal curve;
determining estimated cross-scattering signal intensities of the plurality of detector channels by performing a convolution operation on the first convolution region using a target kernel, wherein the target kernel characterizes scattering contributions from a second source of the second source-detector pair to the first detector; and
determining corrected signal intensities of the plurality of detector channels by performing a cross-scattering correction on the signal intensities of the plurality of detector channels based on the estimated cross-scattering signal intensities of the plurality of detector channels.

15. A non-transitory computer readable medium, comprising a set of instructions for cross-scattering correction for a dual-source or multi-source computed tomography (CT) device, wherein the dual-source or multi-source CT device at least includes a first source-detector pair and a second source-detector pair, and when executed by at least one processor, the set of instructions direct the at least one processor to effectuate a method, the method comprising:
obtaining a detection signal curve of a first detector of the first source-detector pair, wherein the detection signal curve is generated based on signal intensities detected by a plurality of detector channels of the first detector at a scanning angle during a dual-source or multi-source scan of a target subject;
determining a first convolution region based on the detection signal curve;
determining estimated cross-scattering signal intensities of the plurality of detector channels by performing a convolution operation on the first convolution region using a target kernel, wherein the target kernel characterizes scattering contributions from a second source of the second source-detector pair to the first detector; and
determining corrected signal intensities of the plurality of detector channels by performing a cross-scattering correction on the signal intensities of the plurality of detector channels based on the estimated cross-scattering signal intensities of the plurality of detector channels.
